# EUROPEAN PATENT APPLICATION

(11) **EP 2 255 796 A2**
(43) Date of publication of application: **01.12.2010**
(21) Application number: 09711817.8
(22) Date of filing: 23.02.2009
(51) Int. Cl.: A61K 9/52, A61K 9/22, A61K 9/00, A61K 31/165, A61K 31/41, A61K 47/00

(54) **PHARMACEUTICAL PREPARATION**

(30) Priority: 22.02.2008 KR 20080016518
(71) Applicant: HanAll Biopharma Co., Ltd., Daejeon 306-120 (KR)
(72) Inventor: KIM, Sung Wuk, Seongnam-si Gyeonggi-do 463-802 (KR); JUN, Sung Soo, Seongnam-si Gyeonggi-do 463-777 (KR); JO, Young Gwan, Daejeon 305-330 (KR); KOO, Ja Seong, Daejeon 305-756 (KR); SON, Jaw Woon, Suwon-si Gyeonggi-do 442-753 (KR); KIM, Jin Wook, Daejeon 302-120 (KR)
(74) Representative: Pohlman, Sandra M.
(86) International application number: PCT/KR2009/000856
(87) International publication number: WO 2009/104939

(57) **Abstract**

The present invention provides a pharmaceutical preparation including a compartment containing a renin inhibitor as a pharmacologically active ingredient, and a compartment containing an HMG-CoA reductase inhibitor as a pharmacologically active ingredient, wherein one compartment is a prior-release compartment and the other compartment is a delayed-release compartment. The combination preparation of the present invention can deliver a renin inhibitor and an HMG-CoA reductase inhibitor with a time interval at a specific speed, thus reducing undesirable side-effects, improving the drug efficacy and promoting the patient compliance. Further, the pharmaceutical preparation of the present invention has pharmacological, clinical, scientific and economical advantages in the prevention or treatment of metabolic syndromes, cardiovascular diseases, renal diseases and the like, as compared with the complex drug regimens in which medicament ingredients are taken individually or simultaneously.

## Description

### TECHNICAL FIELD

The present invention relates to a pharmaceutical preparation containing a renin inhibitor and a 3-hydroxy-3-methylglutaryl coenzyme A (HMG-CoA) reductase inhibitor.

### BACKGROUND ART

Hyperlipidemia or serum lipid level elevation is related to an increased incidence of cardiovascular diseases and arteriosclerosis. Examples of hyperlipidemia include hypercholesterolemia, familial dysbetalipoproteinemia, diabetic dyslipemia, nephritic dyslipidemia and familial combined hyperlipidemia. Hypercholesterolemia is characterized by the elevation of a low-density lipoprotein (LDL)-cholesterol level and a total cholesterol level in serum, and a lowering of the serum lipid level, especially the LDL-cholesterol level, can lead to decreased pathogenic risk of cardiovascular diseases and delayed progression or degeneration of arteriosclerosis (American Diabetes Association, Diabetic Care, 23 (supple.), S57-565, 2000).

That is, hypercholesterolemia and hyperlipidemia are involved in the early development of atherosclerosis which is characterized by non-uniform distribution of lipid deposits inside arteries including coronary arteries, carotid arteries and peripheral arteries. Further, irregular atheroma distribution is thus characteristic of coronary artery damage and cardiovascular diseases, the risk factors also include the existence of diabetes, gender, smoking, and left ventricular hypertrophy due to complications of hypertension [Wilson et al., Am. J. Cardiol., vol. 59(14), 91G-94G, 1987].

Hypertension frequently coexists with coronary artery diseases and both of them are primary pathogenic factors for the development of cardiac diseases. Expression of these risk factors is potentially due to a common mechanism. Arteriosclerosis, occurring due to hypertension and hyperlipidemia, is a clinical condition which becomes worse when both symptoms are coexisting. Specifically, when blood pressure increases, arteriosclerosis becomes worse, and when arteriosclerosis becomes worse, blood pressure increases, such that arteriosclerosis and hypertension may aggravate symptoms of each other [Am.J.Cardiol; 1987: 59(14), 91-94]. Therefore, it is already well known that it would be beneficial for patients to receive a combination therapy in order to treat such conditions, and such a combination therapy clinically becomes a basic therapeutic strategy.

It is already well known that combined administration of an HMG-CoA reductase inhibitor with a conventional anti-hypertensive drug is advantageous for the treatment of cardiovascular diseases and renal diseases. However, there is no combination drug product for combined use and combined administration of the HMG-CoA reductase inhibitor and the renin inhibitor in the form of a single formulation and furthermore, there is no introduction of a combination composition whose release is controlled taking into consideration pharmacological mechanisms including absorption, distribution and metabolism.

Renin, which is synthesized in the kidney, migrates to blood streams, catalyzes an angiotensin converting enzyme (ACE) cleaving angiotensinogen, which leads to the release of angiotensin-I. Then, angiotensin-I is cleaved in the lung, kidney and other organs, thereby forming angiotensin-II. Angiotensin-II can increase blood pressure directly by arterial vasoconstriction and in association with the sodium-ion-retaining hormone aldosterone, may cause blood pressure elevation, left ventricular hypertrophy, blood vessel hypertrophy, atherosclerosis, renal failure, cerebral stroke and the like.

Inhibition of the enzymatic activity of renin in hypertensive patients brings about a reduction in the formation of angiotensin I. As a result, a smaller amount of angiotensin-II is produced. The reduced concentration of that peptide hormone may be a direct cause of anti-hypertensive effects.

An inhibitor of the enzymatic activity of renin (hereinafter, referred to as "renin inhibitor") developed based on the above-discussed biomechanism can be used for the treatment of diastolic dysfunction and diastolic heart failure by controlling the blood pressure and blood volume, and is widely used as a therapeutic drug for the treatment of hypertension since the renin inhibitor can retard the initiation of left ventricular hypertrophy and consequent heart fibrosis or can reverse the progression thereof through the suppression of angiotensin-II production.

Further, a renin inhibitor is certified to have more authentic hypotensive action and additional effects through single administration thereof as well as by combined administration thereof with a diuretic (such as hydrochlorothiazide), a calcium channel blocker, an angiotensin converting enzyme inhibitor and/or an angiotensin II receptor blocker [J Hypertens 2007; Jan 25(1): 217-26, J Manag Care Pharm 2007; Oct 13 (8 Suppl B): 21-33, Cardiol Rev 2007; Nov-Dec 15(6): 316-23, Am J Hypertens 2006; Feb 24(2): 243-56].

Most of currently available renin inhibitors, such as remikiren, enalkiren, and zankiren, which were developed based on the mechanism of inhibiting the conversion of angiotensinogen into angiotensin-I by inhibiting the cleavage of angiotensinogen through the binding with renin, have a short half-life and a low bioavailability, thus exhibiting poor hypotensive effects [Pharmacol Ther 1994; 61: 325-44, Expert Opin Ther Patents 2003; 13: 589-603, J Cardiovasc Pharmacol 1989; 14: 221-26, Lancet 2006; 368: 1449-56]. Among renin inhibitors, aliskiren has a long half-life of 24 hours and therefore can be administered once a day in spite of a low bioavailability (2% or less) [J Hypertens 2005; 23: 417-26]. Further, aliskiren is an anti-hypertension drug, which has been acknowledged to have hypotensive action and additional effects through single administration thereof as well as by combined administration thereof with a hydrochlorothiazide, a calcium channel blocker, an angiotensin converting enzyme inhibitor and/or an angiotensin-II receptor blocker [J Hypertens 2007; Jan 25(1): 217-26, J Manag Care Pharm 2007; Oct 13 (8 Suppl B): 21-33, Cardiol Rev 2007; Nov-Dec 15(6): 316-23, Am J Hypertens 2006; Feb 24(2): 243-56].

Aliskiren is chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide and is specifically disclosed in EP 678503 A. Preferred is a hemi-fumarate salt thereof [Recent patents on Cardiovascular Drug Discovery 2006; Nov 1(3): 233-40].

Atorvastatin is the most frequently used and typical agent among HMG-CoA reductase inhibitors. Atorvastatin serves to strongly inhibit the catalysis of conversion of an HMG-CoA reductase, 3-hydroxy-3-methylglutaryl coenzyme A (HMG-CoA) into mevalonate, which consequently inhibits the cholesterol biosynthesis in the liver, thus lowering the low-density lipoprotein-cholesterol (LDL-C) level. Due to having such effects, atorvastatin is excellent in the treatment of combined hyperlipidemia and clinically normal arteriosclerotic disorders and the prevention of the progression thereof. Further, it was experimentally demonstrated that the LDL-cholesterol level-lowering activity of atorvastatin is very effective for coronary heart diseases ["Reduction in Cardiovascular Events With Atorvastatin in 2,532 Patients With Type 2 Diabetes" Diabetes Care 200528: 1151-1157, "Different Time Course for Prevention of Coronary and Stroke Events by Atorvastatin in the Anglo-Scandinavian Cardiac Outcomes Trial-Lipid-Lowering Arm (ASCOT-LLA)" The American Journal of Cardiology 2005; 96: 39-44].

It is well known that the HMG-CoA reductase inhibitor is the first-choice drug for the prevention and treatment of heart diseases due to coronary arteriosclerosis, such as angina pectoris and myocardial infarction [Lancet 1995; 346: 750-753, Am J Cardiol 1998; 82: 57T-59T, Am J Cardiol 1995; 76: 107C-112C, Hypertens Res 2003; 26: 699-704, Hypertens Res 2003; 26: 273-280, Br Med Bull 2001; 59: 3-16, Am J Med 1998; 104 (Suppl 1): 6S-8S, Clin Pharmacokinet 2002; 41: 343-370].

Such an action is due to the fact that atorvastatin strongly inhibits HMG-CoA reductase playing a crucial role in the synthesis of cholesterol in the liver and at the same time, atorvastatin exerts an inhibitory action on inflammatory factors [Effects of atorvastatin on inflammation and oxidative stress" Heart and Vessels, 2005, 20(4), 133-136].

In particular, a low dose of atorvastatin reduces inflammation of patients with angina pectoris and a low cholesterol level [Int J Cardiol 2006; 109: 48-52].

The synthesis of lipids in the liver is active after early evening meals, so there has been recommended that the HMG-CoA reductase inhibitor is administered in the early evening [Arterioscler Thromb 11: 816-826, Clinic Pharmacol Ther 40: 338-343]. If the patients receiving the planned angioplasty take atorvastatin in the evening, the endothelial cell function is far more improved as compared to morning administration of atorvastatin. The evening administration of atorvastatin exhibited a greater decrease in total cholesterol level, LDL-cholesterol level and triglyceride level, and showed a higher HDL-cholesterol level [Am J Cardiol 2006; 97: 44-47].

It is already well known that combined use and combined administration of an angiotensin converting enzyme inhibitor or an angiotensin-II receptor blocker with an HMG-CoA reductase inhibitor is advantageous for the treatment of cardiovascular diseases and renal diseases. With regard to aliskiren which is a renin inhibitor, combined use and combined administration of aliskiren with an HMG-CoA reductase inhibitor such as atorvastatin are also expected to have hypotensive action and additional effects.

However, co-administration of aliskiren with atorvastatin leads to an increase in blood aliskiren level, which consequently may cause side effects.

Atorvastatin strongly inhibits the catalysis of conversion of an HMG-CoA reductase, 3-hydroxy-3-methylglutaryl coenzyme A (HMG-CoA) into mevalonate, which consequently inhibits cholesterol biosynthesis in the liver, thus lowering the low-density lipoprotein-cholesterol (LDL-C) level. In order to exhibit such lipid-lowering effects, atorvastatin should work in the liver. Meanwhile, atorvastatin is a drug which is absorbed by the small intestine and enters the liver, metabolized by first-pass metabolism, when it is administered. Atorvastatin is largely metabolized by cytochrome P450 3A4 in the liver, and works in the liver, followed by excretion.

Aliskiren exhibits a peak blood level 1 to 3 hours after administration thereof. However, when high-fat diet is ingested, aliskiren is significantly affected by a diet, such as 71% decrease in bioavailability and 85% decrease in peak blood level.

On the other hand, it has been reported that aliskiren is metabolized by cytochrome P450 3A4 in vitro experiments, although it does not inhibit or induce a hepatic enzyme cytochrome P450.

However, it is known that concurrent administration of aliskiren and atorvastatin exhibits a 50% increase in blood aliskiren level. From the viewpoint of this, simultaneous administration of atorvastatin and aliskiren should be avoided and two drugs should be administered with a time interval of dissolution and absorption thereof within the range that can avoid an interaction [Circulation 2007; 115: e69-e171, US FDA drug approvals PDR (Tekturna [packet insert] East Hanover, NJ: Novartis Pharmaceuticals Corporation; 2007)].

As a combination therapy for improving various disease conditions, a combination therapy containing an HMG-CoA reductase inhibitor and a renin inhibitor has been proposed as follows.

International Patent Publication No. WO 2002/40007 discloses a synergistic combination for treating cardiovascular diseases, using an HMG-CoA reductase inhibitor and a renin inhibitor. Aliskiren is described as a usable renin inhibitor. The above-mentioned invention relating to a combination therapy is a pharmaceutical composition which is based on simple combination of drugs, and therefore is merely a simple combination preparation which does not take into consideration characteristics and absorption principles of individual drugs.

As a result of a variety of extensive and intensive studies and experiments to solve the problems associated with simple combined administration of a renin inhibitor and an HMG-CoA reductase inhibitor, the inventors have completed the present invention as follows.

### DISCLOSURE OF THE INVENTION

### TECHNICAL PROBLEM

Therefore, the present invention is intended to provide a preparation which is capable of minimizing side effects occurring upon combined administration of a renin inhibitor and an HMG-CoA reductase inhibitor, inducing optimum pharmacological effects, obtaining clinical synergistic effects through the administration of drugs at a time point where pharmacological effects of individual drugs are expressed, and enhancing the compliance of patients.

### TECHNICAL SOLUTION

The present invention provides a pharmaceutical preparation including a compartment containing a renin inhibitor as a pharmacologically active ingredient, and a compartment containing a 3-hydroxy-3-methylglutaryl coenzyme A (HMG-CoA) reductase inhibitor as a pharmacologically active ingredient, wherein one compartment is a prior-release compartment and the other compartment is a delayed-release compartment.

As used herein, the term "renin inhibitor" is intended to include an isomer thereof or a pharmaceutically acceptable salt thereof, and the term "HMG-CoA reductase inhibitor" is also intended to include an isomer thereof or a pharmaceutically acceptable salt thereof.

There is no particular limitation to the renin inhibitor as long as it is affected by an HMG-CoA reductase inhibitor. The renin inhibitor may be selected from aliskiren, remikiren, enalkiren, zankiren, detikiren, terlakiren, isomers thereof or pharmaceutically acceptable salts thereof. A pharmaceutically acceptable salt of aliskiren may be aliskiren hemi-fumarate.

The HMG-CoA reductase inhibitor may be selected from atorvastatin, simvastatin, pitavastatin, rosuvastatin, fluvastatin, pravastatin, lovastatin, isomers thereof or pharmaceutically acceptable salts thereof. The isomer of atorvastatin includes (R,R) isomers, (R,S) isomers, (S,S) isomers, (S,R) isomers and racemates thereof.Without particular limitation, preferably, the renin inhibitor in the preparation of the present invention is aliskiren, an isomer thereof or a pharmaceutically acceptable salt thereof (hereinafter, referred to as "aliskiren") and the HMG-CoA reductase inhibitor is atorvastatin, an isomer thereof or a pharmaceutically acceptable salt thereof (hereinafter, referred to as "atorvastatin"); or the renin inhibitor is aliskiren, an isomer thereof or a pharmaceutically acceptable salt thereof (hereinafter, referred to as "aliskiren") and the HMG-CoA reductase inhibitor is simvastatin, an isomer thereof or a pharmaceutically acceptable salt thereof (hereinafter, referred to as "simvastatin"); or the renin inhibitor is aliskiren, an isomer thereof or a pharmaceutically acceptable salt thereof (hereinafter, referred to as "aliskiren") and the HMG-CoA reductase inhibitor is fluvastatin, an isomer thereof or a pharmaceutically acceptable salt thereof (hereinafter, referred to as "fluvastatin"); or the renin inhibitor is aliskiren, an isomer thereof or a pharmaceutically acceptable salt thereof (hereinafter, referred to as "aliskiren") and the HMG-CoA reductase inhibitor is rosuvastatin, an isomer thereof or a pharmaceutically acceptable salt thereof (hereinafter, referred to as "rosuvastatin"); or the renin inhibitor is aliskiren, an isomer thereof or a pharmaceutically acceptable salt thereof (hereinafter, referred to as "aliskiren") and the HMG-CoA reductase inhibitor is pitavastatin, an isomer thereof or a pharmaceutically acceptable salt thereof (hereinafter, referred to as "pitavastatin"); or the renin inhibitor is aliskiren, an isomer thereof or a pharmaceutically acceptable salt thereof (hereinafter, referred to as "aliskiren") and the HMG-CoA reductase inhibitor is pravastatin, an isomer thereof or a pharmaceutically acceptable salt thereof (hereinafter, referred to as "pravastatin"); or the renin inhibitor is aliskiren, an isomer thereof or a pharmaceutically acceptable salt thereof (hereinafter, referred to as "aliskiren") and the HMG-CoA reductase inhibitor is lovastatin, an isomer thereof or a pharmaceutically acceptable salt thereof (hereinafter, referred to as "lovastatin").

The preparation in accordance with the present invention provides more useful therapeutic effects by the provision of a physical compartment for controlling release properties between two pharmacologically active ingredients, thereby improving problems of conventional combined administration or concurrent administration of single drugs.

A pharmacologically active ingredient of the prior-release compartment in the preparation of the present invention is released at a level of more than 80% by weight of a total amount of the pharmacologically active ingredient of the prior-release compartment in the preparation within one hour after the release of the pharmacologically active ingredient is initiated. Preferably, more than 90% by weight of a total amount of the pharmacologically active ingredient in the preparation within one hour.

Further, a pharmacologically active ingredient of the delayed-release compartment in the preparation of the present invention is released at a level of less than 40% by weight of a total amount of the pharmacologically active ingredient of the delayed-release compartment in the preparation by 2 hours after the release of the pharmacologically active ingredient of the prior-release compartment is initiated. Preferably, the pharmacologically active ingredient of the delayed-release compartment is released at a level of less than 20% by weight of a total amount of the pharmacologically active ingredient of the delayed-release compartment by 2 hours after the release of the pharmacologically active ingredient of the prior-release compartment is initiated.

The present invention provides a pharmaceutical preparation wherein the pharmacologically active ingredient of the delayed-release compartment is metabolized in the liver at a time-lag interval of 2 hours after the pharmacologically active ingredient of the prior-release compartment is metabolized.

The preparation of the present invention may contain 1 to 3000 parts by weight of the HMG-CoA reductase inhibitor such as atorvastatin, based on 100 parts by weight of the renin inhibitor such as aliskiren. If a content of the HMG-CoA reductase inhibitor is lower than 1 part by weight, it may be difficult to exhibit significant pharmacological activity as a lipid-lowering agent. On the other hand, if a content of the HMG-CoA reductase inhibitor is higher than 3000 parts by weight, long-term administration is scheduled taking into consideration the nature of a subject disease, which may result in the risk of intrinsic side effects of the drug due to high-dose administration thereof.

A content of the renin inhibitor (such as aliskiren) in the preparation of the present invention may be in the range of 10 to 1000 mg in the pharmaceutical preparation, and preferably 35 to 600 mg.

A content of the HMG-CoA reductase inhibitor (such as atorvastatin) in the preparation of the present invention may be in the range of 0.5 to 160 mg in the pharmaceutical preparation, and preferably 1 to 80 mg.

Hereinafter, individual compartments of the pharmaceutical preparation in accordance with the present invention will be described in more detail.

### 1. Prior-release compartment

The "prior-release compartment" refers to a compartment which is released ahead of the "delayed-release compartment" in the pharmaceutical preparation of the present invention. The prior-release compartment may contain "pharmaceutically acceptable additives", if necessary, in addition to "pharmacologically active ingredients".

### (1) Pharmacologically active ingredients

The pharmacologically active ingredient of the prior-release compartment is 1) a renin inhibitor, or 2) an HMG-CoA reductase inhibitor.

### (2) Pharmaceutically acceptable additives

The preparation of the present invention may further contain commonly used additives such as pharmaceutically acceptable diluent, binder, disintegrant, lubricant, stabilizer, pH-adjusting agent, and solubilizer, within the range where effects of the present invention are not impaired and the release of pharmacologically active ingredients is not impaired.

Examples of the diluent may include starch, microcrystalline cellulose, lactose, glucose, mannitol, alginate, an alkaline earth metal salt, clay, polyethylene glycol, dicalcium phosphate, and a mixture thereof.

In the prior-release compartment of the present invention, a content of the additive is in the range of 0.1 to 300 parts by weight, relative to 1 part by weight of the active ingredient.

Examples of the diluent that can be used in the prior-release compartment of the present invention may include starch, microcrystalline cellulose, lactose, glucose, mannitol, alginate, an alkaline earth metal salt, clay, polyethylene glycol, dicalcium phosphate, and a mixture thereof.

Examples of the binder that can be used in the prior-release compartment of the present invention may include starch, microcrystalline cellulose, highly dispersive silica, mannitol, sucrose, lactose, polyethylene glycol, polyvinylpyrrolidone, hydroxypropylmethylcellulose, hydroxypropylcellulose, natural gum, synthetic gum, copovidone, povidone, gelatin, and a mixture thereof.

Examples of the disintegrant that can be used in the prior-release compartment of the present invention may include starches or modified starches such as sodium starch glycolate, corn starch, potato starch, and pregelatinized starch, clays such as bentonite, montmorillonite, and veegum, celluloses such as microcrystalline cellulose, hydroxypropylcellulose, and carboxymethylcellulose, algins such as sodium alginate, and alginic acid, crosslinked celluloses such as croscarmellose sodium, gums such as guar gum, and xanthan gum, crosslinked polymers such as crosslinked polyvinylpyrrolidone (crospovidone), effervescent agents such as sodium bicarbonate and citric acid, and mixtures thereof.

Examples of the lubricant that can be used in the prior-release compartment of the present invention may include talc, stearic acid, magnesium stearate, calcium stearate, sodium lauryl sulfate, hydrogenated vegetable oil, sodium benzoate, sodium stearyl fumarate, glyceryl behenate, glyceryl monolaurate, glyceryl monostearate, glyceryl palmitostearate and polyethylene glycol.

Examples of the stabilizer that can be used in the prior-release compartment of the present invention may include ascorbic acid, citric acid, butylated hydroxyanisole, butylated hydroxy toluene, and tocopherol derivatives. Further examples of the stabilizer may include alkalizers such as alkali metal salts, alkaline earth metal salts, or mixtures thereof. Preferably, there may be used calcium carbonate, sodium carbonate, sodium hydrogen carbonate, magnesium oxide, magnesium carbonate, and sodium citrate. Examples of the pH-adjusting agent that can be used in the prior-release compartment of the present invention may include acidulants such as acetic acid, adipic acid, ascorbic acid, malic acid, succinic acid, tartaric acid, fumaric acid, and citric acid, and basifying agents such as precipitated calcium carbonate, aqueous ammonia, and meglumine.

Examples of the solubilizer that can be used in the prior-release compartment of the present invention may include sodium lauryl sulfate, polyoxyethylene sorbitan fatty acid ester (such as polysorbate), and docusate sodium.

In addition, the preparation of the present invention may optionally contain pharmaceutically acceptable additives such as various additives selected from a colorant and a fragrance. The range of the additive that can be used in the present invention is not limited to the above-mentioned additives, and the additive may be used in a conventional dose which can be suitably selected by those skilled in the art.

### 2. Delayed-release compartment

In the present invention, the "delayed-release compartment" refers to a compartment whose active ingredient is released at a certain time interval after the release of the active ingredient of the "prior-release compartment". The delayed-release compartment may contain (1) a "pharmacologically active ingredient" and (2) a "release-controlling material" or (3) an osmo-regulator and a semi-permeable membrane coating base, and (4), if necessary, "pharmaceutically acceptable additives".

### (1) Pharmacologically active ingredients

The pharmacologically active ingredient of the delayed-release compartment is 1) a renin inhibitor, or 2) an HMG-CoA reductase inhibitor. Here, the pharmacologically active ingredient of the delayed-release compartment is an ingredient which is not identical with the pharmacologically active ingredient of the prior-release compartment. For instance, when the pharmacologically active ingredient of the prior-release compartment is 1) a renin inhibitor, the pharmacologically active ingredient of the delayed-release compartment is 2) an HMG-CoA reductase inhibitor, or vice versa.

The active ingredient of the delayed-release compartment in the preparation of the present invention is released at a level of less than 40% by weight of a total amount of the active ingredient of the delayed-release compartment in the preparation by 2 hours after the release of the active ingredient of the prior-release compartment is initiated. Preferably, the active ingredient of the delayed-release compartment is released at a level of less than 20% by weight of a total amount of the active ingredient of the delayed-release compartment by 2 hours after the release of the active ingredient of the prior-release compartment is initiated.

### (2) Release-controlling materials

The delayed-release compartment in the pharmaceutical preparation of the present invention may contain at least one release-controlling material selected from an enteric polymer, a water-insoluble polymer, a hydrophobic compound, a hydrophilic polymer and a mixture thereof. A preferred example of the release-controlling material is at least one selected from an enteric polymer, a hydrophilic polymer and a water-insoluble polymer, specifically at least one selected from a water-insoluble polymer, hydroxypropylcellulose, hydroxypropylmethylcellulose, hydroxypropylmethylcellulose phthalate, a methacrylic acid/ethyl acrylate copolymer, and a carboxyvinyl polymer.

In the delayed-release compartment of the present invention, a content of the release-controlling material is in the range of 0.01 to 100 parts by weight relative to 1 part by weight of the active ingredient. If a content of the release-controlling material is lower than 0.01 parts by weight, it may be difficult to achieve a sufficient time-lag. On the other hand, if a content of the release-controlling material is higher than 100 parts by weight, it may be difficult to achieve significant clinical effects due to delayed release of the drug.

In the delayed-release compartment of the present invention, the enteric polymer refers to a polymer which is insoluble or stable under the acidic conditions of less than pH 5, and is dissolved or degraded under the specific pH conditions of pH 5 or higher. The enteric polymer that can be used in the present invention is selected from the group consisting of an enteric cellulose derivative, an enteric acrylic acid copolymer, an enteric maleic acid copolymer, an enteric polyvinyl derivative, and a mixture thereof.

Preferably, the enteric cellulose derivative is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid copolymer, a methacrylic acid/methyl methacrylate copolymer (e.g., Eudragit L 100 or Eudragit S, Degussa, Germany), a methacrylic acid/ethyl acrylate copolymer (e.g., Eudragit L 100-55, Degussa, Germany), a methyl acrylate/methacrylic acid/octyl acrylate copolymer and a mixture thereof; the enteric maleic acid copolymer is at least one selected from a vinylacetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetal phthalate, polyvinylbutyrate phthalate, polyvinylacetacetal phthalate and a mixture thereof.

A content of the enteric polymer may be in the range of 0.1 parts by weight to 20 parts by weight, preferably 0.5 parts by weight to 10 parts by weight relative to 1 part by weight of the active ingredient. If a content of the enteric polymer is lower than 0.1 parts by weight, the enteric polymer may be easily dissolved at a pH of less than 5. On the other hand, if a content of the enteric polymer is higher than 20 parts by weight, this may lead to an unnecessary increase in a total weight of the preparation or excessively delayed release thereof.

In the delayed-release compartment of the present invention, the water-insoluble polymer refers to a pharmaceutically acceptable water-insoluble polymer which controls the release of drug. The water-insoluble polymer that can be used in the present invention is preferably at least one selected from the group consisting of polyvinyl acetate, a polymethacrylate copolymer, a poly(ethyl acrylate, methyl methacrylate) copolymer, a poly(ethyl acrylate, methyl methacrylate, trimethylaminoethyl methacrylate) copolymer, ethylcellulose, cellulose ester, cellulose ether, cellulose acylate, cellulose diacylate, cellulose triacylate, cellulose acetate, cellulose diacetate, cellulose triacetate and a mixture thereof. A content of the water-insoluble polymer may be in the range of 0.1 parts by weight to 30 parts by weight, preferably 0.5 parts by weight to 20 parts by weight relative to 1 part by weight of the active ingredient. If a content of the water-insoluble polymer is lower than 0.1 parts by weight, release of the drug may not be controlled. On the other hand, if a content of the water-insoluble polymer is higher than 30 parts by weight, release of the drug may be excessively delayed.

In the delayed-release compartment of the present invention, the hydrophobic compound refers to a pharmaceutically acceptable water-insoluble material which controls the release of a drug. The hydrophobic compound that can be used in the present invention may be at least one selected from the group consisting of fatty acid and fatty acid ester, fatty acid alcohol, wax, inorganic material, and a mixture thereof. Preferably, the fatty acid or fatty acid ester is at least one selected from glyceryl palmitostearate, glyceryl stearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof.

A content of the hydrophobic compound may be in the range of 0.1 parts by weight to 20 parts by weight, preferably 0.5 parts by weight to 10 parts by weight relative to 1 part by weight of the active ingredient. If a content of the hydrophobic compound is lower than 0.1 parts by weight, release of the drug may be not controlled. On the other hand, if a content of the hydrophobic compound is higher than 20 parts by weight, release of the drug may be excessively delayed.

In the delayed-release compartment of the present invention, the hydrophilic polymer refers to a pharmaceutically acceptable water-soluble polymer which controls the release of a drug. The hydrophilic polymer that can be used in the present invention may be at least one selected from the group consisting of saccharide, a cellulose derivative, gum, a protein, a polyvinyl derivative, a polymethacrylate copolymer, a polyethylene derivative, a carboxyvinyl copolymer and a mixture thereof. Preferably, the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose, hydroxypropylcellulose, hydroxymethylcellulose, hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the polymethacrylate copolymer is at least one selected from a poly(butyl methacrylate, (2-dimethylaminoethyl)methacrylate, methyl methacrylate) copolymer, a poly(methacrylic acid, methyl methacrylate) copolymer, a poly(methacrylic acid, ethyl acrylate) copolymer and a mixture thereof; the polyethylene derivative is at least one selected from polyethylene glycol, polyethylene oxide and a mixture thereof; and the carboxyvinyl polymer is carbomer.

A content of the hydrophilic polymer may be in the range of 0.05 parts by weight to 30 parts by weight, preferably 0.5 to 20 parts by weight relative to 1 part by weight of the active ingredient. If a content of the hydrophilic polymer is lower than 0.05 parts by weight, release of the drug may be not controlled. On the other hand, if a content of the hydrophilic polymer is higher than 30 parts by weight, release of the drug may be excessively delayed.

The delayed-release compartment in the pharmaceutical preparation of the present invention contains at least one release-controlling material selected from an enteric polymer, a water-insoluble polymer, a hydrophobic compound, a hydrophilic polymer and a mixture thereof, and preferably contains an enteric polymer and a hydrophilic polymer, or contains a water-insoluble polymer.

Further, in the delayed-release compartment of the present invention, a preferred example of the water-insoluble polymer which is a release-controlling material is at least one selected from polyvinylacetate, a poly(ethyl acrylate, methyl methacrylate, trimethylaminoethyl methacrylate) copolymer, ethyl cellulose, cellulose acetate and a mixture thereof.

In the delayed-release compartment of the present invention, a content of the release-controlling material is in the range of 0.01 to 100 parts by weight relative to 1 part by weight of the active ingredient. If a content of the release-controlling material is lower than 0.01 parts by weight, it may be difficult to achieve a sufficient time-lag. On the other hand, if a content of the release-controlling material is higher than 100 parts by weight, this may result in no release of the drug or an excessively long time-lag.

### (3) Osmo-regulator and semi-permeable membrane coating base

The delayed-release compartment of the present invention may be a compartment which contains an osmo-regulator and is coated by a semi-permeable membrane coating base.

In the delayed-release compartment of the present invention, the osmo-regulator is preferably at least one selected from the group consisting of magnesium sulfate, magnesium chloride, sodium chloride, lithium chloride, potassium sulfate, sodium sulfate, lithium sulfate and a mixture thereof.

A content of the osmo-regulator may be in the range of 0.01 parts by weight to 10 parts by weight, preferably 0.05 parts by weight to 0.5 parts by weight relative to 1 part by weight of the active ingredient. If a content of the osmo-regulator is lower than 0.01 parts by weight, it may be difficult to achieve a sufficient chronotherapeutic release. On the other hand, if a content of the osmo-regulator is higher than 10 parts by weight, it may be difficult to achieve significant clinical effects due to delayed release of the drug.

In the present invention, the semi-permeable membrane coating base is a material which is compounded in a coating layer of the pharmaceutical preparation and refers to a material used to form a membrane through which some ingredients can pass but other ingredients cannot pass. The semi-permeable coating base in the present invention may employ the above-mentioned water-insoluble polymers.

A content of the semi-permeable membrane coating base may be in the range of 0.01 parts by weight to 10 parts by weight, preferably 0.05 parts by weight to 1.25 parts by weight relative to 1 part by weight of the active ingredient. If a content of the semi-permeable membrane coating base is lower than 0.01 parts by weight, it may be difficult to achieve a sufficient time-lag. On the other hand, if a content of the semi-permeable membrane coating base is higher than 10 parts by weight, there is a problem associated with no release of the drug or an excessively long time-lag.

### (4) Pharmaceutically acceptable additives

In addition to (2) the release-controlling material, the preparation of the present invention may further contain commonly used additives such as pharmaceutically acceptable diluent, binder, disintegrant, lubricant, pH-adjusting agent, anti-foaming agent, and solubilizer, within the range where the effects of the present invention are not impaired and within the range where delayed-release properties are not compromised.

Examples of the diluent that can be used in the present invention may include starch, microcrystalline cellulose, lactose, glucose, mannitol, alginate, an alkaline earth metal salt, clay, polyethylene glycol, dicalcium phosphate, and a mixture thereof. Examples of the binder that can be used in the present invention include starch, microcrystalline cellulose, highly dispersive silica, mannitol, sucrose, lactose, polyethylene glycol, polyvinylpyrrolidone, hydroxypropylmethylcellulose, hydroxypropylcellulose, natural gum, synthetic gum, copovidone, povidone, gelatin, and a mixture thereof.

Examples of the disintegrant that can be used in the present invention may include starches or modified starches such as sodium starch glycolate, corn starch, potato starch, and pregelatinized starch, clays such as bentonite, montmorillonite, and veegum, celluloses such as microcrystalline cellulose, hydroxypropylcellulose, and carboxymethylcellulose, algins such as sodium alginate, and alginic acid, crosslinked celluloses such as croscarmellose sodium, gums such as guar gum, and xanthan gum, crosslinked polymers such as crosslinked polyvinylpyrrolidone (crospovidone), effervescent agents such as sodium bicarbonate and citric acid, and mixtures thereof.

Examples of the lubricant that can be used in the present invention may include talc, stearic acid, magnesium stearate, calcium stearate, sodium lauryl sulfate, hydrogenated vegetable oil, sodium benzoate, colloidal silicon dioxide, sodium stearyl fumarate, glyceryl behenate, glyceryl monolaurate, glyceryl monostearate, glyceryl palmitostearate and polyethylene glycol.

As the pharmaceutically acceptable additive, examples of the pH-control agent that can be used in the present invention may include acidulants such as acetic acid, adipic acid, ascorbic acid, malic acid, succinic acid, tartaric acid, fumaric acid, and citric acid, and basifying agents such as precipitated calcium carbonate, aqueous ammonia, and meglumine.

As the pharmaceutically acceptable additive of the present invention, examples of the anti-foaming agent may include dimethicone, oleyl alcohol, propylene glycol alginate, and simethicone (such as simethicone emulsion).

As the pharmaceutically acceptable additive of the present invention, examples of the solubilizer may include sodium lauryl sulfate, polyoxyethylene sorbitan fatty acid ester (such as polysorbate), and docusate sodium.

In addition, the preparation of the present invention may contain pharmaceutically acceptable additives such as various additives selected from a colorant and a fragrance. The range of the additives that can be used in the present invention is not limited to the above-mentioned additives, and the additive may be used in a conventional dose which can be suitably selected by those skilled in the art.

Further, in the delayed-release preparation of the present invention, as a binding solvent and a solvent for the delayed-release additive, there may be employed purified water, ethanol, methylene chloride, and the like. More preferred are purified water, and ethanol.

In the pharmaceutically acceptable additive of the present invention, the range of the usable additive is not limited to the above-mentioned additive, and the additive may be used in a conventional dose which can be suitably selected by those skilled in the art.

The pharmaceutical preparation of the present invention can be prepared into various formulations, for example, tablets (such as uncoated tablets, coated tablets, multi-layered tablets, or press coated tablets), powders, granules, or capsules.

The pharmaceutical preparation of the present invention may be in the form of a two-phase matrix tablet which is obtained by uniformly mixing a delayed-release compartment and a prior-release compartment, followed by compression.

Further, the pharmaceutical preparation of the present invention may be in the form of a film-coated tablet including a tablet consisting of a delayed-release compartment and a film-coated layer consisting of a prior-release compartment enclosing the exterior of the tablet, whereby atorvastatin of the film-coated layer is first released as the film-coated layer is dissolved.

Further, the pharmaceutical preparation of the present invention may be in the form of a multi-layered tablet having a multi-layered structure of a delayed-release compartment and a prior-release compartment, each compartment of which is obtained by mixing the granules constituting the delayed-release compartment and the prior-release compartment with pharmaceutical additives, and compressing the mixture into a double-layered or triple-layered tablet, using a multiple tablet press. The resulting preparation is a tablet for oral administration which was formulated to achieve the prior-release and delayed-release of drugs according to individual layers.

Further, the pharmaceutical preparation of the present invention may be in the form of a press coated tablet including an inner core formed of a delayed-release compartment and an outer layer formed of a prior-release compartment enclosing the outer surface of the inner core. The press coated tablet may be an osmotic press coated tablet. The osmotic press coated tablet is a formulation wherein the tablet mix is compressed into a tablet in a manner that an osmo-regulator is incorporated for the delayed-release of a drug, the tablet surface is coated with a semi-permeable membrane coating base to prepare an inner core, a granule constituting the prior-release compartment is mixed with a pharmaceutical additive to prepare an outer layer, followed by compression to form a formulation having a delayed-release inner core and a prior-release layer enclosing the surface of the inner core.

The pharmaceutical preparation of the present invention may be in the form of a capsule containing a particle, granule, pellet, or tablet formed of a delayed-release compartment and a particle, granule, pellet, or tablet formed of a prior-release compartment.

The preparation of the present invention may further include a coating layer on the outside of the delayed-release compartment and/or the prior-release compartment. That is, the surface of particles, granules, pellets, or tablets formed of the delayed-release compartment and/or the prior-release compartment may be coated for the purpose of controlled release of drugs or stability of the preparation. Further, the pharmaceutical preparation of the present invention may be in the form of a kit including a delayed-release compartment and a prior-release compartment. Specifically, the kit includes (a) prior-release compartment, (b) a delayed-release compartment, and (c) a container for filling the prior-release compartment and the delayed-release compartment. The kit can be prepared in the form of a kit wherein a particle, granule, pellet, or tablet constituting the prior-release compartment is prepared, a granule, pellet, or tablet constituting the delayed-release compartment is additionally prepared, and the thus prepared two release compartments are filled in a foil, blister, or bottle to prepare a dosage form for concurrent administration of different drugs.

The preparation of the present invention may be a preparation in the form of an uncoated tablet without further coating, or otherwise, if necessary, may be provided in the form of a coated tablet further including a coating layer formed on the outside of the preparation. The formation of a coating layer can provide a preparation which is capable of further securing stability of pharmacologically active ingredients.

A method for forming the coating layer may be suitably selected by a skilled person in the art, from among methods capable of forming a film-like coating layer on the surface of the tablet layer, such as a fluidized-bed coating method and a pan coating method. Preferably, a pan coating method may be used.

The coating layer may be prepared by using a film-forming agent, a film-forming aid or a mixture thereof. Specifically, the film-forming agent may be cellulose derivatives such as hydroxypropylmethylcellulose and hydroxypropylcellulose, saccharide derivatives, polyvinyl derivatives, waxes, fats, gelatin and mixtures thereof, and the film-forming aid may be polyethylene glycol, ethylcellulose, glyceride, titanium oxide, talc, diethyl phthalate and mixtures thereof.

A content of the coating layer may be in the range of 0.5 to 15% by weight (% w/w) based on the total weight of the tablet.

If a content of the coating layer is lower than 0.5% by weight, it may be difficult to achieve the protection of products, and the stability thereof depending on formulations. On the other hand, if a content of the coating layer is higher than 15% by weight, release profiles of pharmacologically active ingredients may be affected.

Further, the present invention provides a pharmaceutical preparation for evening administration in accordance with the present invention.

The preparation of the present invention can be administered once a day, particularly in the evening time (17 to 23 o'clock), thus providing maximized effects of individual pharmacologically active ingredients and minimized side effects. Since biosynthesis of cholesterol is active during the night, it is effective to administer an HMG-CoA reductase inhibitor such as atorvastatin in the evening. In addition, a renin inhibitor such as aliskiren has duration of action of 24 hours, so evening administration thereof is effective against a peak blood pressure which reaches in the morning. Therefore, these two drugs should be administered in the evening, but there is a risk of side effects due to an interaction therebetween upon combined administration of single drugs. Administration of a combination preparation in accordance with the present invention reduces the risk of side effects, potentiates the drug efficacy, and improves the compliance of patients.

Therefore, the preparation of the present invention may be intended for evening administration.

Further, the delayed-release compartment in accordance with the present invention may also be administered simultaneously with a commercially available pharmacologically active ingredient of the prior-release compartment.

The present invention provides a method for preventing and treating at least one disease selected from a metabolic syndrome, a cardiovascular disease and a kidney disease, including administering the pharmaceutical preparation of the present invention to a mammal including a human.

The cardiovascular disease includes hypertension and complications thereof of people suffering from a metabolic syndrome with combined manifestation of hypertension, or diabetes, obesity, hyperlipidemia, and coronary artery diseases.

The pharmaceutical preparation of the present invention can be preferably formulated into a desired dosage form depending on individual diseases or ingredients, by an appropriate method known in the art, for example, using the principle of the chronotherapy as disclosed in Chrontherapeutics (2003, Peter Redfern, PhP), specifically by a method including the following steps.

Step 1 is a step of obtaining a delayed-release granule or tablet by subjecting a pharmacologically active ingredient of the delayed-release compartment and one or two release-controlling materials selected from the group consisting of an enteric polymer, a water-insoluble polymer, a hydrophobic compound, and a hydrophilic polymer together with a pharmaceutically acceptable conventional additive to mixing, kneading, drying, granulation or coating, and compression, or of obtaining a delayed-release granule or tablet by subjecting the pharmacologically active ingredient and an osmo-regulator together with a conventional pharmaceutically acceptable additive to mixing, kneading, drying, granulation or compression, followed by coating with a semi-permeable membrane coating base.

Step 2 is a step of obtaining a prior-release granule or tablet by subjecting a pharmacologically active ingredient of the prior-release compartment together with a conventional pharmaceutically acceptable additive to conventional processes for producing oral solid preparations, for example, mixing, kneading, drying, granulation or coating, and compression.

Step 3 is a step of obtaining a preparation for oral administration by mixing the granule or tablet obtained in each of Steps 1 and 2 with a pharmaceutically acceptable excipient and either compressing the mixture into a tablet or filling the mixture in a capsule for oral administration.

Step 1 may be carried out after Step 2, or Step 1 may be carried out simultaneously with Step 2.

The pharmaceutical preparation of the present invention can be prepared according to the above procedure, and a formulation method of Step 3 will be described in more detail hereinafter, but the present invention is not limited thereto.

### 1. Preparation of two-phase matrix tablets

The particles or granules prepared in Step 1 are optionally coated with a release-controlling material and then mixed with the granules prepared in Step 2, followed by compression into uniform weight, thereby preparing tablets. The resulting tablets may be film-coated for the purpose of improving the stability or shape, if necessary.

### 2. Preparation of film-coated tablets containing pharmacologically active ingredients

The coated tablets or granules prepared in Step 1 are optionally coated with a release-controlling material and dried, followed by compression into uniform weight and optionally further coating to prepare tablets. In addition, a pharmacologically active ingredient of the prior-release compartment is dissolved and dispersed in a water-soluble film coating solution and is coated on the outer layer of the tablets obtained in Step 1 to thereby prepare oral film-coated tablets containing pharmacologically active ingredients in the film coating.

### 3. Preparation of multi-layered tablets

The granules prepared in Step 1 are optionally coated with a release-controlling material, and dried. The dried granules are compressed with the granules prepared in Step 2 by using a multi-layered tablet press, thereby obtaining a double-layered tablet. According to the formulation design or if necessary, a triple or more multi-layered tablet may also be prepared by further adding a release adjuvant layer on the double-layered tablet. A coated multi-layered tablet may be prepared by coating the multi-layered tablet.

### 4. Preparation of press-coated tablets

The coated tablets or granules prepared in Step 1 are optionally coated with a release-controlling material and dried, followed by compression into uniform weight. The resulting granules are used as an inner core optionally after performing further coating, and compressed with the granules prepared in Step 2 by using a press-coated tablet press, thereby providing press-coated tablets in where the surface of the tablet of Step 1 is enclosed by the prior-release layer. Coated press-coated tablets may be prepared by coating the press-coated tablets.

### 5. Preparation of capsules (containing granules or tablets)

The granules prepared in Step 1 are optionally coated with a release-controlling material, and dried. The dried granules together with the granules prepared in Step 2 may be placed in a capsule filling machine, and filled in a capsule having a given size at an effective amount of each main ingredient, thereby preparing a capsule.

### 6. Preparation of capsules (pellets)

(1) A pharmacologically active ingredient of the delayed-release compartment, a release-controlling material, and if necessary, pharmaceutically acceptable additives are dissolved or suspended in water, an organic solvent, or a mixed solvent. This solution or suspension is coated on sugar spheres and dried, and if necessary, coated with one or more release-controlling materials dissolved in water, an organic solvent, or a mixed solvent, followed by drying. The mixture may be mixed with the granules prepared in Step 2 or the tablets obtained in Step 3 and then filled in capsules using a capsule filling machine, thereby preparing capsules.
(2) A pharmacologically active ingredient of the prior-release compartment and pharmaceutically acceptable additives may be dissolved or suspended in water, an organic solvent or a mixed solvent, coated on sugar spheres, followed by drying, and mixed with the controlled-release pellets of Section (1) containing a pharmacologically active ingredient of the delayed-release compartment and filled in capsules using a capsule filling machine to prepare capsules.

### 7. Preparation of kit

The preparation of Step 1 containing a pharmacologically active ingredient of the delayed-release compartment and the preparation of Step 2 containing a pharmacologically active ingredient of the prior-release compartment may be filled in a foil, blister, or bottle to prepare a kit for concurrent administration of different drugs.

### ADVANTAGEOUS EFFECTS

The combination preparation of the present invention can deliver a renin inhibitor and an HMG-CoA reductase inhibitor with a time interval at a specific speed, thus reducing undesirable side-effects, improving the drug efficacy and promoting the patient compliance. Further, the pharmaceutical preparation of the present invention has pharmacological, clinical, scientific and economical advantages in the prevention or treatment of metabolic syndromes, cardiovascular diseases, renal diseases and the like, as compared with the complex drug regimens in which medicament ingredients are taken individually or simultaneously.

### DESCRIPTION OF THE DRAWINGS

FIG. 1 is a graph showing the comparative dissolution profiles of an aliskiren-atorvastatin preparation prepared in Example 1, and the aliskiren and atorvastatin ingredients of single drugs, Tekturna and Lipitor, as control drugs.
FIG. 2 is a graph showing the comparative dissolution profiles of an aliskiren-atorvastatin preparation prepared in Example 4, and the aliskiren and atorvastatin ingredients of single drugs, Tekturna and Lipitor, as control drugs.
FIG. 3 is a graph showing the comparative dissolution profiles of an aliskiren-simvastatin multi-layered tablet prepared in Example 10, and the aliskiren and simvastatin ingredients of single drugs, Tekturna and Zocor, as control drugs.
FIG. 4 is a graph showing the comparative dissolution profiles of an aliskiren-simvastatin multi-layered tablet prepared in Example 9, and the aliskiren and simvastatin ingredients of single drugs, Tekturna and Zocor, as control drugs.
FIG. 5 is a graph showing the dissolution profiles of Examples 12 to 15.
FIG. 6 is a graph showing the dissolution profiles of Examples 14, and 16 to 18.
FIG. 7 is a graph showing the comparative dissolution profiles of an aliskiren-pravastatin sodium capsule (pellet-tablet) preparation prepared in Example 21, and the aliskiren and pravastatin ingredients of single drugs, Tekturna and Pravachol, as control drugs.
FIG. 8 is a graph showing the comparative dissolution profiles of an aliskiren-lovastatin combination controlled-release capsule (granule-granule) preparation prepared in Example 23, and the aliskiren and lovastatin ingredients of single drugs, Tekturna and Mevacor, as control drugs.

### MODE FOR INVENTION

Now, the present invention will be described in more detail with reference to the following Examples. These examples are provided only for illustrating the present invention and should not be construed as limiting the scope and spirit of the present invention.

### Example 1: Preparation of aliskiren-atorvastatin two-phase matrix tablets

According to the ingredient compositions and contents shown in Table 1 below, the preparation was carried out as follows.

### 1) Preparation of aliskiren delayed-release granules

Aliskiren hemi-fumarate, microcrystalline cellulose, crospovidone, and sodium chloride were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, cellulose acetate (acetyl group 32%), cellulose acetate (acetyl group 39.8%), and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany) to prepare aliskiren hemi-fumarate delayed-release granules.

### 2) Preparation of atorvastatin prior-release granules

Atorvastatin calcium, and microcrystalline cellulose, lactose, corn starch and sodium starch glycolate (as excipients) were sieved through a No. 35 sieve and mixed in a high-speed mixer. Meanwhile, hydroxypropylcellulose and citric acid were dissolved in water to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole was added and mixed with the sieved material.

### 3) Compression and coating

The aliskiren hemi-fumarate delayed-release granules of Process 1) and the atorvastatin calcium prior-release granules of Process 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example 2: Preparation of aliskiren-simvastatin two-phase matrix tablets

According to the ingredient compositions and contents shown in Table 1 below, the preparation was carried out as follows.

### 1) Preparation of aliskiren delayed-release granules

Aliskiren hemi-fumarate and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, hydroxypropylmethylcellulose phthalate and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany) to prepare aliskiren hemi-fumarate delayed-release granules.

### 2) Preparation of simvastatin prior-release granules

Simvastatin, and microcrystalline cellulose, lactose, corn starch and sodium starch glycolate (as excipients) were sieved through a No. 35 sieve and mixed in a high-speed mixer. Meanwhile, hydroxypropylcellulose and citric acid were dissolved in water to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole was added and mixed with the sieved material.

### 3) Compression and coating

The aliskiren hemi-fumarate delayed-release granules of Process 1) and the simvastatin prior-release granules of Process 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example 3: Preparation of aliskiren-fluvastatin two-phase matrix tablets

According to the ingredient compositions and contents shown in Table 1 below, the preparation was carried out as follows.

### 1) Preparation of aliskiren delayed-release granules

Aliskiren hemi-fumarate and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, Kollicoat SR 30D was dissolved and dispersed in 220 mg of ethanol and 980 mg of methylene chloride to prepare a coating solution which was then coated on the granules in a fluidized bed coater (GPCG-1: Glatt, Germany) to prepare aliskiren hemi-fumarate delayed-release granules.

### 2) Preparation of fluvastatin prior-release granules

Fluvastatin sodium, and microcrystalline cellulose, D-mannitol and sodium starch glycolate (as excipients) were sieved through a No. 35 sieve and mixed in a high-speed mixer. Meanwhile, hydroxypropylcellulose and citric acid were dissolved in water to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole was added and mixed with the sieved material.

### 3) Compression and coating

The aliskiren hemi-fumarate delayed-release granules of Process 1) and the fluvastatin calcium prior-release granules of Process 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example 4: Preparation of atorvastatin-aliskiren two-phase matrix tablets

According to the ingredient compositions and contents shown in Table 1 below, the preparation was carried out as follows.

### 1) Preparation of atorvastatin delayed-release granules

Atorvastatin calcium, microcrystalline cellulose, crospovidone, and sodium chloride were sieved through a No. 35 sieve and mixed in a high-speed mixer for 5 minutes to prepare a mixture. Meanwhile, polyvinylpyrrolidone and citric acid were dissolved in purified water to prepare a binding solution, followed by kneading, granulation and drying. The dried material was placed in a fluidized bed coater. Meanwhile, cellulose acetate (acetyl group: 32%), cellulose acetate (acetyl group: 39.8%), and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany). To the coated granules was added butylated hydroxyanisole, followed by mixing to prepare atorvastatin delayed-release granules.

### 2) Preparation of aliskiren prior-release granules

Aliskiren hemi-fumarate, and microcrystalline cellulose, lactose, corn starch and sodium starch glycolate (as excipients) were sieved through a No. 35 sieve and mixed in a high-speed mixer. Meanwhile, hydroxypropylcellulose was dissolved in water to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 3) Compression and coating

The atorvastatin calcium delayed-release granules of Process 1) and the aliskiren hemi-fumarate prior-release granules of Process 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example 5: Preparation of simvastatin-aliskiren two-phase matrix tablets

According to the ingredient compositions and contents shown in Table 1 below, the preparation was carried out as follows.

### 1) Preparation of simvastatin delayed-release granules

Simvastatin and microcrystalline cellulose were sieved through a No. 35 sieve and mixed in a high-speed mixer for 5 minutes to prepare a mixture. Meanwhile, polyvinylpyrrolidone and citric acid were dissolved in purified water to prepare a binding solution, followed by kneading, granulation and drying. The dried material was placed in a fluidized bed coater. Meanwhile, hydroxypropylmethylcellulose phthalate and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany). To the coated material was added butylated hydroxyanisole, followed by mixing to prepare simvastatin delayed-release granules.

### 2) Preparation of aliskiren prior-release granules

Aliskiren hemi-fumarate, and microcrystalline cellulose, D-mannitol and sodium starch glycolate (as excipients) were sieved through a No. 35 sieve and mixed in a high-speed mixer. Meanwhile, hydroxypropylcellulose was dissolved in water to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 3) Compression and coating

The simvastatin calcium delayed-release granules of Process 1) and the aliskiren hemi-fumarate prior-release granules of Process 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example 6: Preparation of fluvastatin-aliskiren two-phase matrix tablets

According to the ingredient compositions and contents shown in Table 1 below, the preparation was carried out as follows.

### 1) Preparation of fluvastatin delayed-release granules

Fluvastatin sodium and microcrystalline cellulose were sieved through a No. 35 sieve and mixed in a high-speed mixer for 5 minutes to prepare a mixture. Meanwhile, polyvinylpyrrolidone and citric acid were dissolved in purified water to prepare a binding solution, followed by kneading, granulation and drying. The dried material was placed in a fluidized bed coater. Meanwhile, Kollicoat SR 30D was dissolved and dispersed in 220 mg of ethanol and 980 mg of methylene chloride to prepare a coating solution which was then coated on the granules in a fluidized bed coater (GPCG-1: Glatt, Germany). To the coated material was added butylated hydroxyanisole, followed by mixing to prepare fluvastatin delayed-release granules.

### 2) Preparation of aliskiren prior-release granules

Aliskiren hemi-fumarate, and microcrystalline cellulose, lactose, corn starch and sodium starch glycolate (as excipients) were sieved through a No. 35 sieve and mixed in a high-speed mixer. Meanwhile, hydroxypropylcellulose was dissolved in water to prepare a binding solution. The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 3) Compression and coating

The fluvastatin calcium delayed-release granules of Process 1) and the aliskiren hemi-fumarate prior-release granules of Process 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example 7: Preparation of atorvastatin-aliskiren multi-layered tablets

According to the ingredient compositions and contents shown in Table 1 below, the preparation was carried out as follows.

### 1) Preparation of atorvastatin delayed-release granules

Atorvastatin calcium, and microcrystalline cellulose, crospovidone and sodium chloride (as excipients) were sieved through a No. 35 sieve and mixed in a high-speed mixer for 5 minutes to prepare a mixture. Meanwhile, polyvinylpyrrolidone and citric acid were dissolved in purified water to prepare a binding solution, followed by kneading, granulation and drying. The dried material was placed in a fluidized bed coater. Meanwhile, cellulose acetate (acetyl group: 32%), cellulose acetate (acetyl group: 39.8%), and hydroxypropylmethylcellulose were dissolved in 220 mg of ethanol and 980 mg of methylene chloride to prepare a solution which was then coated on the granules in a fluidized bed coater (GPCG-1: Glatt, Germany). After completion of the coating process, butylated hydroxyanisole was added and mixed the coated granules, and magnesium stearate was added thereto, followed by final mixing in a double cone mixer for 4 minutes to prepare an atorvastatin delayed-release layer.

### 2) Preparation of aliskiren prior-release granules

Aliskiren hemi-fumarate, and microcrystalline cellulose and D-mannitol (as excipients) were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. The sieved material was mixed with sodium starch glycolate, and magnesium stearate was added thereto, followed by final mixing in a double cone mixer.

### 3) Compression and coating

Tablet compression was carried out using a multi-layered tablet press (MRC-37T: Sejong, South Korea). The atorvastatin calcium-containing composition of Process 1) was placed in a first powder feeder, and the aliskiren hemi-fumarate delayed-release layer composition of Process 2) was placed in a second powder feeder. The compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing sustained-release tablets in the form of a multi-layered tablet.

### Example 8: Preparation of atorvastatin-aliskiren multi-layered tablets

According to the ingredient compositions and contents shown in Table 1 below, the preparation was carried out as follows.

### 1) Preparation of atorvastatin delayed-release granules

Atorvastatin calcium and microcrystalline cellulose were sieved through a No. 35 sieve and mixed in a high-speed mixer for 5 minutes to prepare a mixture. Meanwhile, polyvinylpyrrolidone and citric acid were dissolved in purified water to prepare a binding solution, followed by kneading, granulation and drying. The dried material was placed in a fluidized bed coater. Meanwhile, hydroxypropylmethylcellulose phthalate and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany). To the coated material was added butylated hydroxyanisole, followed by mixing, and magnesium stearate was added thereto, followed by final mixing in a double cone mixer for 4 minutes to prepare simvastatin delayed-release granules.

### 2) Preparation of aliskiren prior-release granules

Aliskiren hemi-fumarate, and microcrystalline cellulose and D-mannitol (as excipients) were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve and the granules were dried in a hot-water dryer at 60°C. After completion of the drying process, the granules were sieved again through a No. 20 sieve. The sieved material was mixed with sodium starch glycolate, and magnesium stearate was added thereto, followed by final mixing in a double cone mixer.

### 3) Compression and coating

Tablet compression was carried out using a multi-layered tablet press (MRC-37T: Sejong, South Korea). The atorvastatin calcium-containing composition of Process 1) was placed in a first powder feeder, and the aliskiren hemi-fumarate delayed-release layer composition of Process 2) was placed in a second powder feeder. The compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing sustained-release tablets in the form of a multi-layered tablet.

### Example 9: Preparation of simvastatin-aliskiren multi-layered tablets

According to the ingredient compositions and contents shown in Table 1 below, the preparation was carried out as follows.

### 1) Preparation of simvastatin delayed-release granules

Simvastatin and microcrystalline cellulose were sieved through a No. 35 sieve and mixed in a high-speed mixer for 5 minutes to prepare a mixture. Meanwhile, polyvinylpyrrolidone and citric acid were dissolved in purified water to prepare a binding solution, followed by kneading, granulation and drying. The dried material was placed in a fluidized bed coater. Meanwhile, Kollicoat SR 30D was dissolved and dispersed in 220 mg of ethanol and 980 mg of methylene chloride to prepare a coating solution which was then coated on the granules in a fluidized bed coater (GPCG-1: Glatt, Germany). To the coated material was added butylated hydroxyanisole, followed by mixing, and magnesium stearate was added thereto, followed by final mixing in a double cone mixer for 4 minutes to prepare simvastatin delayed-release granules.

### 2) Preparation of aliskiren prior-release granules

Aliskiren hemi-fumarate, and microcrystalline cellulose and D-mannitol (as excipients) were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. The sieved material was mixed with sodium starch glycolate, and magnesium stearate was added thereto, followed by final mixing in a double cone mixer.

### 3) Compression and coating

Tablet compression was carried out using a multi-layered tablet press (MRC-37T: Sejong, South Korea). The simvastatin-containing composition of Process 1) was placed in a first powder feeder, and the aliskiren hemi-fumarate delayed-release layer composition of Process 2) was placed in a second powder feeder. The compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing sustained-release tablets in the form of a multi-layered tablet.

### Example 10: Preparation of aliskiren-simvastatin multi-layered tablets

According to the ingredient compositions and contents shown in Table 2 below, the preparation was carried out as follows.

### 1) Preparation of aliskiren delayed-release granules

Aliskiren hemi-fumarate, and microcrystalline cellulose, crospovidone and sodium chloride (as excipients) were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, cellulose acetate (acetyl group: 32%), cellulose acetate (acetyl group: 39.8%), and hydroxypropylmethylcellulose were dissolved in 220 mg of ethanol and 980 mg of methylene chloride to prepare a solution which was then coated on the granules in a fluidized bed coater (GPCG-1: Glatt, Germany). After completion of the coating process, magnesium stearate was added thereto, followed by mixing for 4 minutes to prepare aliskiren hemi-fumarate delayed-release granules.

### 2) Preparation of simvastatin prior-release granules

Simvastatin, and microcrystalline cellulose and D-mannitol (as excipients) were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. The sieved material was mixed with butylated hydroxyanisole and sodium starch glycolate, and magnesium stearate was added thereto, followed by final mixing in a double cone mixer.

### 3) Compression and coating

Tablet compression was carried out using a multi-layered tablet press (MRC-37T: Sejong, South Korea). The simvastatin-containing composition of Process 2) was placed in a first powder feeder, and the aliskiren hemi-fumarate delayed-release layer composition of Process 1) was placed in a second powder feeder. The compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing sustained-release tablets in the form of a multi-layered tablet.

### Example 11: Preparation of aliskiren-simvastatin multi-layered tablets

According to the ingredient compositions and contents shown in Table 2 below, the preparation was carried out as follows.

### 1) Preparation of aliskiren delayed-release granules

As shown in Table 2 below, aliskiren hemi-fumarate and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, hydroxypropylmethylcellulose phthalate and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany). After completion of the coating process, magnesium stearate was added thereto, followed by mixing for 4 minutes to prepare aliskiren hemi-fumarate delayed-release granules.

### 2) Preparation of rosuvastatin prior-release granules

As shown in Table 2 below, rosuvastatin, and microcrystalline cellulose and D-mannitol (as excipients) were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. The sieved material was mixed with butylated hydroxyanisole and sodium starch glycolate, and magnesium stearate was added thereto, followed by final mixing in a double cone mixer.

### 3) Compression and coating

Tablet compression was carried out using a multi-layered tablet press (MRC-37T: Sejong, South Korea). The rosuvastatin-containing composition of Process 2) was placed in a first powder feeder, and the aliskiren hemi-fumarate delayed-release layer composition of Process 1) was placed in a second powder feeder. The compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing sustained-release tablets in the form of multi-layered tablet.

### Example 12: Preparation of aliskiren-atorvastatin multi-layered tablets

According to the ingredient compositions and contents shown in Table 2 below, the preparation was carried out as follows.

### 1) Preparation of aliskiren delayed-release granules

Aliskiren hemi-fumarate, microcrystalline cellulose and pregelatinized starch were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, colloidal silicon dioxide, ethylcellulose and methacrylic acid copolymer type C were dissolved in 220 mg of ethanol and 980 mg of methylene chloride to prepare a solution which was then coated on the granules in a fluidized bed coater (GPCG-1: Glatt, Germany). After completion of the coating process, magnesium stearate was added thereto, followed by mixing for 4 minutes to prepare aliskiren hemi-fumarate delayed-release granules.

### 2) Preparation of atorvastatin prior-release granules

Atorvastatin, and microcrystalline cellulose, lactose and corn starch (as excipients) were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. The sieved material was mixed with butylated hydroxyanisole and sodium starch glycolate, and magnesium stearate was added thereto, followed by final mixing in a double cone mixer.

### 3) Compression and coating

Tablet compression was carried out using a multi-layered tablet press (MRC-37T: Sejong, South Korea). The atorvastatin-containing composition of Process 2) was placed in a first powder feeder, and the aliskiren hemi-fumarate delayed-release granules of Process 1) were placed in a second powder feeder. The compositions in the feeders were compressed into tablets under such conditions that the interlayer incorporation can be minimized. Meanwhile, hydroxypropylmethylcellulose 2910, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing sustained-release tablets in the form of a multi-layered tablet.

### Example 13: Preparation of aliskiren-atorvastatin multi-layered tablets

According to the ingredient compositions and contents shown in Table 2 below, the preparation was carried out as follows.

### 1) Preparation of aliskiren delayed-release granules

Based on the composition and content of Table 2, aliskiren delayed-release granules were prepared in the same manner as in the preparation of aliskiren delayed-release granules of Example 12.

### 2) Preparation of atorvastatin prior-release granules

Based on the composition and content of Table 2, aliskiren delayed-release granules were prepared in the same manner as in the preparation of atorvastatin prior-release granules of Example 12

### 3) Compression and coating

The compression and coating were carried out in the same manner as in the compression and coating of Example 12.

### Example 14: Preparation of aliskiren-atorvastatin multi-layered tablets

According to the ingredient compositions and contents shown in Table 2 below, the preparation was carried out as follows.

### 1) Preparation of aliskiren delayed-release granules

Aliskiren hemi-fumarate, microcrystalline cellulose, pregelatinized starch, and crospovidone were sieved through a No. 35 sieve and mixed in a high-speed mixer for 5 minutes to prepare a mixture. Meanwhile, polyvinylpyrrolidone was dissolved in purified water to prepare a binding solution, followed by kneading, granulation and drying. The dried material was placed in a fluidized bed coater. Meanwhile, colloidal silicon dioxide, ethylcellulose and methacrylic acid copolymer type C were dissolved in 220 mg of ethanol and 980 mg of methylene chloride to prepare a solution which was then coated on the granules in a fluidized bed coater (GPCG-1: Glatt, Germany). After completion of the coating process, magnesium stearate was added thereto, followed by mixing for 4 minutes to prepare aliskiren hemi-fumarate delayed-release granules.

### 2) Preparation of atorvastatin prior-release granules

Based on the ingredients and contents of Table 2, atorvastatin prior-release granules were prepared in the same manner as in Process 2) of Example 12.

### 3) Compression and coating

The compression and coating were carried out in the same manner as in 3) Compression and coating in Example 12.

### Example 15: Preparation of aliskiren-atorvastatin multi-layered tablets

According to the ingredient compositions and contents shown in Table 2 below, the preparation was carried out as follows.

### 1) Preparation of aliskiren delayed-release granules

Based on the composition and content of Table 2, aliskiren delayed-release granules were prepared in the same manner as in 1) Preparation of aliskiren delayed-release granules in Example 12.

### 2) Preparation of atorvastatin prior-release granules

Based on the composition and content of Table 2, aliskiren delayed-release granules were prepared in the same manner as in 2) Preparation of atorvastatin prior-release granules in Example 12.

### 3) Compression and coating

The compression and coating were carried out in the same manner as in 3) Compression and coating in Example 12.

### Example 16: Preparation of aliskiren-atorvastatin multi-layered tablets

According to the ingredient compositions and contents shown in Table 2 below, the preparation was carried out as follows.

### 1) Preparation of aliskiren delayed-release granules

Based on the composition and content of Table 2, aliskiren delayed-release granules were prepared in the same manner as in 1) Preparation of aliskiren delayed-release granules in

### Example 14.

### 2) Preparation of atorvastatin prior-release granules

Based on the ingredient and content of Table 2, aliskiren delayed-release granules were prepared in the same manner as in 2) Preparation of atorvastatin prior-release granules in Example 12.

### 3) Compression and coating

The compression and coating were carried out in the same manner as in 3) Compression and coating in Example 12.

### Example 17: Preparation of aliskiren-atorvastatin multi-layered tablets

According to the ingredient compositions and contents shown in Table 2 below, the preparation was carried out as follows.

### 1) Preparation of aliskiren delayed-release granules

Based on the composition and content of Table 2, aliskiren delayed-release granules were prepared in the same manner as in 1) Preparation of aliskiren delayed-release granules in Example 14.

### 2) Preparation of atorvastatin prior-release layer

Based on the composition and content of Table 2, aliskiren delayed-release granules were prepared in the same manner as in 2) Preparation of atorvastatin prior-release layer in Example 12.

### 3) Compression and coating

The compression and coating were carried out in the same manner as in 3) Compression and coating in Example 12.

### Example 18: Preparation of aliskiren-atorvastatin multi-layered tablets

According to the ingredient compositions and contents shown in Table 2 below, the preparation was carried out as follows.

### 1) Preparation of aliskiren delayed-release granules

Based on the composition and content of Table 2, aliskiren delayed-release granules were prepared in the same manner as in 1) Preparation of aliskiren delayed-release granules in Example 14.

### 2) Preparation of atorvastatin prior-release layer

Based on the composition and content of Table 2, aliskiren delayed-release granules were prepared in the same manner as in 2) Preparation of atorvastatin prior-release layer in Example 12.

### 3) Compression and coating

The compression and coating were carried out in the same manner as in 3) Compression and coating in Example 12.

### Example 19: Preparation of aliskiren-pitavastatin capsules (pellets-granules)

According to the ingredient compositions and contents shown in Table 3 below, the preparation was carried out as follows.

### 1) Preparation of aliskiren delayed-release pellets

Sugar seeds (sugar spheres) were sieved through a No. 35 sieve and placed in a fluidized bed granulator (GPCG 1: Glatt). Meanwhile, hydroxypropylmethylcellulose and aliskiren hemi-fumarate were dissolved in water and ethanol to prepare a binding solution which was then sprayed thereon to form aliskiren hemi-fumarate-containing pellets, followed by drying. A solution of hydroxypropylmethylcellulose phthalate in 220 mg of ethanol and 980 mg of methylene chloride was sprayed on the pellets to prepare aliskiren hemi-fumarate delayed-release pellets.

### 2) Preparation of pitavastatin prior-release granules

As shown in Table 5 below, pitavastatin calcium, microcrystalline cellulose, and D-mannitol were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole was added to the sieved material, followed by final mixing in a double cone mixer.

### 3) Mixing and capsule filling

The final compositions of Processes 1) and 2) were mixed in a double cone mixer. To the mixture was added sodium starch glycolate, followed by mixing in a double cone mixer. Magnesium stearate was added and finally mixed with the mixture. The final mixture was placed in a powder feeder and then filled in capsules using a capsule filling machine (SF 40N, Sejong Machinery Co., Ltd., South Korea), thereby preparing a controlled-release preparation in the form of a capsule.

### Example 20: Preparation of simvastatin-aliskiren capsules (pellets-granules)

According to the ingredient compositions and contents shown in Table 3 below, the preparation was carried out as follows.

### 1) Preparation of simvastatin delayed-release pellets

Sugar seeds (sugar spheres) were sieved through a No. 35 sieve and placed in a fluidized bed granulator (GPCG 1: Glatt). Meanwhile, hydroxypropylmethylcellulose, citric acid and simvastatin were dissolved in water and ethanol to prepare a binding solution which was then sprayed thereon to form aliskiren hemi-fumarate-containing pellets, followed by drying. A solution of hydroxypropylmethylcellulose phthalate in 220 mg of ethanol and 980 mg of methylene chloride was sprayed on the pellets to prepare HMG-CoA reductase inhibitor delayed-release pellets.

### 2) Preparation of aliskiren granules

Aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 3) Mixing and capsule filling

The final compositions of Processes 1) and 2) were mixed in a double cone mixer. To the mixture was added sodium starch glycolate, followed by mixing in a double cone mixer. Magnesium stearate was added to the mixture, followed by final mixing. The final mixture was placed in a powder feeder, and then filled in capsules using a capsule filling machine, thereby preparing a controlled-release preparation in the form of a capsule.

### Example 21: Preparation of aliskiren-pravastatin capsules (pellets-tablets)

According to the ingredient compositions and contents shown in Table 3 below, the preparation was carried out as follows.

### 1) Preparation of aliskiren delayed-release pellets

Sugar seeds (sugar spheres) were sieved through a No. 35 sieve and placed in a fluidized bed granulator (GPCG 1: Glatt). Meanwhile, hydroxypropylmethylcellulose and aliskiren hemi-fumarate were dissolved in water and ethanol to prepare a binding solution which was then sprayed thereon to form aliskiren hemi-fumarate-containing pellets, followed by drying. A solution of hydroxypropylmethylcellulose phthalate in 220 mg of ethanol and 980 mg of methylene chloride was sprayed on the pellets to prepare aliskiren hemi-fumarate delayed-release pellets.

### 2) Preparation of pravastatin prior-release tablets

As shown in Table 5, pravastatin sodium, microcrystalline cellulose, and D-mannitol were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole and sodium starch glycolate were added to the sieved material, followed by mixing in a double cone mixer. Magnesium stearate was added to the mixture, followed by final mixing. The final mixture was compressed into tablets using a rotary tablet press (MRC-33: Sejong, South Korea).

### 3) Capsule filling

The final compositions of Processes 1) and 2) were filled in capsules using a capsule filling machine, thereby preparing a preparation in the form of a capsule.

### Example 22: Preparation of pitavastatin-aliskiren capsules (pellets-tablets)

According to the ingredient compositions and contents shown in Table 3 below, the preparation was carried out as follows.

### 1) Preparation of pitavastatin delayed-release pellets

Sugar seeds (sugar spheres) were sieved through a No. 35 sieve and placed in a fluidized bed granulator (GPCG 1: Glatt). Meanwhile, hydroxypropylmethylcellulose, citric acid and pitavastatin calcium were dissolved in water and ethanol to prepare a binding solution which was then sprayed thereon to form HMG-CoA reductase inhibitor-containing pellets, followed by drying. A solution of hydroxypropylmethylcellulose phthalate in 220 mg of ethanol and 980 mg of methylene chloride was sprayed on the pellets to prepare HMG-CoA reductase inhibitor delayed-release pellets.

### 2) Preparation of aliskiren prior-release tablets

As shown in Table 5, aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. Magnesium stearate was added to the mixture, followed by final mixing. The final mixture was compressed into tablets using a rotary tablet press (MRC-33: Sejong, South Korea).

### 3) Capsule filling

The final compositions of Processes 1) and 2) were filled in capsules using a capsule filling machine, thereby preparing a controlled-release preparation in the form of a capsule.

### Example 23: Preparation of aliskiren-lovastatin capsules (granules-granules)

According to the ingredient compositions and contents shown in Table 3 below, the preparation was carried out as follows.

### 1) Preparation of aliskiren delayed-release granules

Aliskiren hemi-fumarate and microcrystalline cellulose were sieved through a No. 35 sieve and mixed in a high-speed mixer. Kollicoat SR30D was kneaded with the mixture of main ingredients. After completion of the kneading process, the kneaded material was granulated using an oscillator with a No. 18 sieve, and the granules were dried in a hot-water dryer at 60°C. After completion of the drying process, the dried material was sieved again through a No. 20 sieve to prepare aliskiren hemi-fumarate delayed-release granules.

### 2) Preparation of lovastatin prior-release granules

Lovastatin, microcrystalline cellulose, and D-mannitol were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole was added and mixed with the sieved material.

### 3) Mixing and capsule filling

The final compositions of Processes 1) and 2) were mixed in a double cone mixer. To the mixture was added sodium starch glycolate, followed by mixing in a double cone mixer. Magnesium stearate was added to the mixture, followed by final mixing. The final mixture was placed in a powder feeder, and then filled in capsules using a capsule filling machine, thereby preparing a controlled-release preparation in the form of a capsule.

### Example 24: Preparation of rosuvastatin-aliskiren capsules (granules-granules)

According to the ingredient compositions and contents shown in Table 3 below, the preparation was carried out as follows.

### 1) Preparation of rosuvastatin delayed-release granules

Rosuvastatin and microcrystalline cellulose were sieved through a No. 35 sieve and mixed in a high-speed mixer. Kollicoat SR30D and citric acid were kneaded with the mixture of main ingredients. After completion of the kneading process, the kneaded material was granulated using an oscillator with a No. 18 sieve, and the granules were dried in a hot-water dryer at 60°C. After completion of the drying process, the dried material was sieved again through a No. 20 sieve to prepare rosuvastatin delayed-release granules.

### 2) Preparation of aliskiren prior-release granules

Aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 3) Mixing and capsule filling

The final compositions of Processes 1) and 2) were mixed in a double cone mixer. To the mixture was added sodium starch glycolate, followed by mixing in a double cone mixer. Magnesium stearate was added to the mixture, followed by final mixing. The final mixture was placed in a powder feeder, and then filled in capsules using a capsule filling machine, thereby preparing a controlled-release preparation in the form of a capsule.

### Example 25: Preparation of aliskiren-atorvastatin capsules (granules-tablets)

According to the ingredient compositions and contents shown in Table 3 below, the preparation was carried out as follows.

### 1) Preparation of aliskiren delayed-release granules

Aliskiren hemi-fumarate and microcrystalline cellulose were sieved through a No. 35 sieve and placed in a fluidized bed granulator (GPCG 1: Glatt), followed by mixing. Meanwhile, hydroxypropylmethylcellulose was dissolved in water to prepare a binding solution which was then sprayed thereon to form aliskiren hemi-fumarate-containing granules, followed by drying. Onto the granules was sprayed a solution of hydroxypropylmethylcellulose phthalate in 220 mg of ethanol and 980 mg of methylene chloride, thereby preparing aliskiren hemi-fumarate delayed-release granules.

### 2) Preparation of atorvastatin prior-release tablets

Atorvastatin calcium, microcrystalline cellulose, and D-mannitol were sieved through a No. 35 sieve and mixed in a high-speed mixer. Meanwhile, hydroxypropylcellulose and citric acid were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Butylated hydroxyanisole and sodium starch glycolate were added to the sieved material, followed by mixing in a double cone mixer. Magnesium stearate was added to the mixture, followed by final mixing. The final mixture was compressed into tablets using a rotary tablet press (MRC-33: Sejong, South Korea).

### 3) Capsule filling

The final compositions of Processes 1) and 2) were filled in capsules using a capsule filling machine, thereby preparing a controlled-release preparation in the form of a capsule.

### Example 26: Preparation of fluvastatin-aliskiren capsules (granules-tablets)

According to the ingredient compositions and contents shown in Table 3 below, the preparation was carried out as follows.

### 1) Preparation of fluvastatin delayed-release granules

Fluvastatin sodium and microcrystalline cellulose were sieved through a No. 35 sieve and placed in a fluidized bed granulator (GPCG 1: Glatt), followed by mixing. Meanwhile, hydroxypropylmethylcellulose and citric acid were dissolved in water to prepare a binding solution which was then sprayed thereon to form HMG-CoA reductase inhibitor-containing granules, followed by drying. Onto the granules was sprayed a solution of hydroxypropylmethylcellulose phthalate in 220 mg of ethanol and 980 mg of methylene chloride, thereby preparing HMG-CoA reductase inhibitor delayed-release granules.

### 2) Preparation of aliskiren prior-release tablet

Aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. Magnesium stearate was added to the mixture, followed by final mixing. The final mixture was compressed into tablets using a rotary tablet press (MRC-33: Sejong, South Korea).

### 3) Capsule filling

The final compositions of Processes 1) and 2) were filled in capsules using a capsule filling machine, thereby preparing a controlled-release preparation in the form of a capsule.

### Example 27: Preparation of simvastatin-aliskiren press-coated tablets

According to the ingredient compositions and contents shown in Table 3 below, the preparation was carried out as follows.

### 1) Preparation of simvastatin delayed-release core tablets

Simvastatin, microcrystalline cellulose, and pregelatinized starch were sieved through a No. 35 sieve and mixed in a high-speed mixer for 5 minutes. Meanwhile, polyvinylpyrrolidone and citric acid were dissolved in water to prepare a binding solution which was then sprayed thereon to form HMG-CoA reductase inhibitor-containing granules, followed by drying. To the granule mixture was added magnesium stearate, followed by final mixing for 4 minutes. The final mixture was compressed into tablets using a rotary tablet press (MRC-33: Sejong, South Korea). The resulting tablets are used as an inner core. Meanwhile, hydroxypropylmethylcellulose and a methacrylic acid copolymer (type C2) were dissolved and dispersed in 132 mg of ethanol and 33 mg of purified water to prepare a coating solution. The compressed core tablets were coated with the coating solution in a Hi-coater (SFC-30N: Sejong, South Korea) to form a delayed-release inner core.

### 2) Preparation of aliskiren prior-release granules

Aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. Magnesium stearate was added to the mixture, followed by final mixing.

### 3) Compression and coating

Using press-coated tablet press (RUD-1: Kilian), press-coated tablets were prepared including the -coated simvastatin core tablet as an inner core and the aliskiren hemi-fumarate-containing composition as an outer layer. Meanwhile, hydroxypropylmethylcellulose 2910, titanium oxide, and talc were dissolved and dispersed in 132 mg of ethanol and 33 mg of purified water to prepare a coating solution. The compressed press-coated tablets were coated with the coating solution in a Hi-coater (SFC-30N: Sejong, South Korea) to form a film-coated layer, thereby preparing press-coated tablets.

### Example 28: Aliskiren-simvastatin blister package kits

### 1) Preparation of aliskiren delayed-release granules

Aliskiren delayed-release granules were prepared according to the same composition and content as in the preparation of aliskiren delayed-release granules in Example 10.

### 2) Preparation of simvastatin prior-release granules

Simvastatin prior-release granules were prepared according to the same composition and content as in the preparation of simvastatin prior-release granules in Example 10.

### 3) Compression and packaging

The granules of Processes 1) and 2) were compressed into tablets using a rotary tablet press (MRC-33: Sejong, South Korea), and were packed in a blister package container such that they can be simultaneously administered.

### Example 29: Aliskiren-atorvastatin blister package kits

### 1) Preparation of atorvastatin delayed-release granules

Atorvastatin delayed-release granules were prepared according to the same composition and content as in 1) Preparation of atorvastatin delayed-release granules in Example 7.

### 2) Preparation of aliskiren prior-release granules

Aliskiren prior-release granules were prepared according to the same composition and content as in 2) Preparation of aliskiren prior-release granules in Example 7.

### 3) Compression and packaging

The granules of Processes 1) and 2) were compressed into tablets using a rotary tablet press (MRC-33: Sejong, South Korea), and were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

### Examples 30 and 31: Preparation of atorvastatin-aliskiren two-phase matrix tablets

### 1) Preparation of atorvastatin prior-release granules

For Example 30, according to the contents given in Table 4, atorvastatin, and microcrystalline cellulose (Vivapur, JRS), corn starch and precipitated calcium carbonate (Hwail Pharma Co., Ltd., South Korea) (as excipients) were sieved through a No. 35 sieve and mixed in a high-speed mixer (Lab. Pharma Mixer P, Diosna, Germany). Meanwhile, hydroxypropylcellulose (Klucel, BASF) was dissolved in water (50 mg) to prepare a binding solution. The binding solution and the mixture of main ingredients were placed in a high-speed mixer (Lab. Pharma Mixer P, Diosna, Germany), followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve (KYK-60, KoreaMedi Co., Ltd., South Korea).

For Example 31, according to the compositions and contents given in Table 4, prior-release granules were prepared in the same manner as in the above-mentioned preparation of prior-release granules.

### 2) Preparation of aliskiren delayed-release granules

For Example 30, according to the contents given in Table 4, aliskiren, lactose, D-mannitol (Pearlitol 160C, Roquette), and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water to prepare a binding solution which was then sprayed thereon to form granules, followed by drying. A solution of Eudragit RS PO (5% w/w) in a 1:1 mixture of ethanol and methylene chloride was sprayed and coated on the granules.

For Example 31, according to the compositions and contents given in Table 4, delayed-release granules were prepared in the same manner as in the above-mentioned preparation of delayed-release granules.

### 3) Compression and coating

For both of Examples 30 and 31, the compression and coating were carried out according to the following procedure.

The final products of Processes 1) and 2) were first mixed in a double cone mixer (Dasan Pharmatech, South Korea). To the first mixture was added sodium starch glycolate, followed by secondary mixing in a double cone mixer. Magnesium stearate was added to the secondary mixture, followed by final mixing.

The final mixture was compressed into tablets using a rotary tablet press (MRC-33, Sejong Pharmatech Co., Ltd., South Korea). Meanwhile, hydroxypropylmethylcellulose 2910 (HP-55, Shin-Etsu), hydroxypropylcellulose, titanium oxide, and talc were dissolved and dispersed in a 8:2 mixed solution of ethanol and purified water to prepare a coating solution (20%w/w). The compressed tablets were coated with the coating solution in a Hi-coater (SFC-30N, Sejong Pharmatech Co., Ltd., South Korea) to form a film-coated layer, thereby preparing two-phase matrix tablets.

### Examples 32 and 33: Preparation of atorvastatin-aliskiren multi-layered tablets

### 1) Preparation of atorvastatin prior-release granules

For Example 32, according to the contents given in Table 4, atorvastatin, and microcrystalline cellulose, corn starch and precipitated calcium carbonate (as excipients) were sieved through a No. 35 sieve and mixed in a high-speed mixer (Lab. Pharma Mixer P, Diosna, Germany). Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea). The sieved material was mixed with sodium starch glycolate, and magnesium stearate was added thereto, followed by final mixing in a double cone mixer (Dasan Pharmatech, South Korea).

For Example 33, according to the compositions and contents given in Table 4, prior-release granule semi-finished products were prepared in the same manner as in the above-mentioned preparation of prior-release granules.

### 2) Preparation of aliskiren delayed-release granules

For Example 32, according to the contents given in Table 4, aliskiren, lactose, D-mannitol, and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water (50 mg) to prepare a binding solution which was then sprayed thereon to form granules, followed by drying. A solution of hydroxypropylmethylcellulose phthalate (5% w/w) in a 1:1 mixture of ethanol and methylene chloride was sprayed and coated on the granules. Magnesium stearate was added thereto, followed by final mixing in a double cone mixer (Dasan Pharmatech, South Korea).

For Example 33, according to the compositions and contents given in Table 4, granule semi-finished products of the delayed-release layer were prepared in the same manner as in the above-mentioned preparation of delayed-release granules.

### 3) Compression and coating

For both of Examples 32 and 33, the compression and coating were carried out according to the following procedure.

Tablet compression was carried out using a multi-layered tablet press (MRC-37T, Sejong Pharmatech Co., Ltd., South Korea). The atorvastatin prior-release granule-containing composition was placed in a first powder feeder, and the aliskiren delayed-release granule-containing composition was placed in a second powder feeder. The compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in an 8:2 mixture of ethanol and purified water to prepare a coating solution (20%w/w).

The compressed tablets were coated with the coating solution in a Hi-coater (SFC-30N, Sejong Pharmatech Co., Ltd., South Korea) to form a film-coated layer, thereby preparing a preparation in the form of a multi-layered tablet.

### Examples 34 and 35: Preparation of atorvastatin-aliskiren capsules (granules-pellets)

### 1) Preparation of atorvastatin prior-release granules

For Example 34, according to the contents given in Table 4, atorvastatin, microcrystalline cellulose and corn starch were sieved through a No. 35 sieve and mixed in a high-speed mixer (Lab. Pharma Mixer P, Diosna, Germany). Meanwhile, hydroxypropylcellulose and meglumine (Sigma) were dissolved in water (50 mg) to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea).

For Example 35, according to the compositions and contents given in Table 4, granule semi-finished products of the prior-release layer were prepared in the same manner as in the above-mentioned preparation of prior-release granules.

### 2) Preparation of aliskiren delayed-release pellets

For Example 34, according to the contents given in Table 4, a mixture of aliskiren, D-mannitol, microcrystalline cellulose, hydroxypropylmethylcellulose, and citric acid was sieved through a No. 35 sieve. The mixture was dispersed in a 1:1 mixture of ethanol and methylene chloride to prepare a dispersion (20%w/w) which was then used as a drug layer coating solution.

Meanwhile, sugar spheres were placed in a fluidized bed coater (GPCG-1, Glatt, Germany), and the drug coating solution was sprayed and coated thereon. Then, a hydroxypropylmethylcellulose phthalate solution (5% w/w) was further coated thereon to prepare aliskiren delayed-release pellets.

For Example 35, according to the compositions and contents given in Table 4, pellet of the delayed-release layer were prepared in the same manner as in the above-mentioned preparation of delayed-release pellets.

### 3) Mixing and capsule filling

For both of Examples 34 and 35, the mixing and capsule filling were carried out according to the following procedure.

The final products of Processes 1) and 2) were first mixed in a double cone mixer (Dasan Pharmatech, South Korea). To the first mixture was added sodium starch glycolate, followed by secondary mixing in a double cone mixer. Magnesium stearate was added to the secondary mixture, followed by final mixing.

The final mixture was placed in a powder feeder, and then filled in capsules using a capsule filling machine (SF-40N, Sejong Pharmatech Co., Ltd., South Korea), thereby preparing capsules.

### Examples 36 and 37: Preparation of atorvastatin-aliskiren capsules (tablets-pellets)

### 1) Preparation of atorvastatin prior-release tablets

For Example 36, according to the contents given in Table 4, atorvastatin, microcrystalline cellulose, corn starch, and precipitated calcium carbonate were sieved through a No. 35 sieve and mixed in a high-speed mixer (Lab. Pharma Mixer P, Diosna, Germany). Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea). Sodium starch glycolate was added to the sieved material, followed by mixing in a double cone mixer (Dasan Pharmatech, South Korea). Magnesium stearate was added to the mixture, followed by final mixing. The final mixture was compressed into tablets using a rotary tablet press (MRC-33, Sejong Pharmatech Co., Ltd., South Korea).

For Example 37, according to the compositions and contents given in Table 4, prior-release tablets were prepared in the same manner as in the above-mentioned preparation of prior-release tablets.

### 2) Preparation of aliskiren delayed-release pellets

For Example 36, according to the contents given in Table 4, a mixture of aliskiren, D-mannitol, microcrystalline cellulose, hydroxypropylmethylcellulose, and citric acid was sieved through a No. 35 sieve. The mixture was dispersed in a 1:1 mixture of ethanol and methylene chloride to prepare a dispersion (20%w/w) which was then used as a drug layer coating solution.

Meanwhile, sugar spheres were placed in a fluidized bed coater (GPCG-1, Glatt, Germany), and the drug coating solution was sprayed and coated thereon. Then, a hydroxypropylmethylcellulose phthalate solution (5% w/w) was further coated thereon to prepare aliskiren delayed-release pellets which were then placed in a double cone mixer and magnesium stearate was added thereto, followed by final mixing.

For Example 37, according to the compositions and contents given in Table 4, pellets of the delayed-release layer were prepared in the same manner as in the above-mentioned preparation of delayed-release pellets.

### 3) Capsule filling

For both of Examples 36 and 37, the capsule filling was carried out according to the following procedure.

The final products of Processes 1) and 2) were filled in capsules using a capsule filling machine (SF-40N, Sejong Pharmatech Co., Ltd., South Korea), thereby preparing capsules.

### Examples 38 and 39: Preparation of atorvastatin-aliskiren capsules (granules-granules)

### 1) Preparation of atorvastatin prior-release granules

For Example 38, according to the contents given in Table 5, atorvastatin, microcrystalline cellulose and D-mannitol were sieved through a No. 35 sieve and mixed in a high-speed mixer (Lab. Pharma Mixer P, Diosna, Germany). Meanwhile, hydroxypropylcellulose and meglumine were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea).

For Example 39, according to the compositions and contents given in Table 5, prior-release granules were prepared in the same manner as in the above-mentioned preparation of prior-release granules.

### 2) Preparation of aliskiren delayed-release granules

For Example 38, according to the contents given in Table 5, aliskiren, D-mannitol and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water to prepare a binding solution which was then sprayed thereon to form granules, followed by drying. Then, Kollicoat SR30D was sprayed and coated on the granules.

For Example 39, according to the compositions and contents given in Table 5, delayed-release granules were prepared in the same manner as in the above-mentioned preparation of delayed-release granules.

### 3) Mixing and capsule filling

For both of Examples 38 and 39, the mixing and capsule filling were carried out according to the following procedure.

The final products of Processes 1) and 2) were first mixed in a double cone mixer (Dasan Pharmatech, South Korea). To the first mixture was added sodium starch glycolate, followed by secondary mixing in a double cone mixer. Magnesium stearate was added to the secondary mixture, followed by final mixing. The final mixture was placed in a powder feeder, and then filled in capsules using a capsule filling machine (SF-40N, Sejong Pharmatech Co., Ltd., South Korea), thereby preparing a controlled-release preparation in the form of a capsule.

### Examples 40 and 41: Preparation of atorvastatin-aliskiren capsules (granules-tablets)

### 1) Preparation of atorvastatin prior-release granules

For Example 40, according to the contents given in Table 5, atorvastatin, microcrystalline cellulose, D-mannitol, and precipitated calcium carbonate were sieved through a No. 35 sieve and mixed in a high-speed mixer (Lab. Pharma Mixer P, Diosna, Germany). Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea).

For Example 41, according to the compositions and contents given in Table 5, granule semi-finished products of the prior-release layer were prepared in the same manner as in the above-mentioned preparation of prior-release granules.

### 2) Preparation of aliskiren delayed-release coated tablets

For Example 40, according to the contents given in Table 5, aliskiren, D-mannitol, and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed granulator. Meanwhile, hydroxypropylmethylcellulose was dissolved in water to prepare a binding solution which was then sprayed thereon to form granules, followed by drying. Then, powdered Carbomer 71G was added to the granules, and magnesium stearate was added thereto, followed by final mixing in a double cone mixer (Dasan Pharmatech, South Korea).

The final mixture was compressed into tablets having a hardness of 7 to 9kp, a thickness of 3.0mm, and a diameter of 5.5mm, using a rotary tablet press (MRC-33, Sejong Pharmatech Co., Ltd., South Korea) at 30 rpm. Then, a hydroxypropylmethylcellulose phthalate solution (5% w/w) was coated thereon to prepare aliskiren delayed-release coated tablets.

For Example 41, according to the compositions and contents given in Table 5, delayed-release coated tablets were prepared in the same manner as in the above-mentioned preparation of delayed-release coated tablets.

### 3) Capsule filling

For both of Examples 40 and 41, the capsule filling was carried out according to the following procedure.

The final products of Processes 1) and 2) were filled in capsules using a capsule filling machine (SF-40N, Sejong Pharmatech Co., Ltd., South Korea), thereby preparing capsules.

### Examples 42 and 43: Preparation of atorvastatin-aliskiren capsules (tablets-tablets)

### 1) Preparation of atorvastatin prior-release tablets

For Example 42, according to the contents given in Table 5, atorvastatin, microcrystalline cellulose, and corn starch were sieved through a No. 35 sieve and mixed in a high-speed mixer (Lab. Pharma Mixer P, Diosna, Germany). Meanwhile, hydroxypropylcellulose and meglumine were dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea). Sodium starch glycolate was added to the sieved material, followed by mixing in a double cone mixer (Dasan Pharmatech, South Korea). Magnesium stearate was added to the mixture, followed by final mixing. The final mixture was compressed into tablets using a rotary tablet press (MRC-33, Sejong Pharmatech Co., Ltd., South Korea).

For Example 43, according to the compositions and contents given in Table 4, prior-release tablets were prepared in the same manner as in the above-mentioned preparation of prior-release tablets.

### 2) Preparation of aliskiren delayed-release coated tablets

For Example 42, according to the contents given in Table 5, aliskiren, D-mannitol, and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed granulator. Meanwhile, hydroxypropylmethylcellulose and citric acid were dissolved in water to prepare a binding solution which was then sprayed thereon to form granules, followed by drying. Then, powdered Carbomer 71 G was added to the granules, and magnesium stearate was added thereto, followed by final mixing in a double cone mixer (Dasan Pharmatech, South Korea).

The final mixture was compressed into tablets having a hardness of 7 to 9kp, a thickness of 3.0mm, and a diameter of 5.5mm, using a rotary tablet press (MRC-33, Sejong Pharmatech Co., Ltd., South Korea) at 30 rpm. Then, a hydroxypropylmethylcellulose phthalate solution (5% w/w) was coated thereon to prepare an aliskiren delayed-release coated inner core.

For Example 43, according to the compositions and contents given in Table 5, delayed-release coated tablets were prepared in the same manner as in the above-mentioned preparation of delayed-release coated tablets.

### 3) Capsule filling

For both of Example 42 and 43, the capsule filling was carried out according to the following procedure.

The final products of Processes 1) and 2) were filled in capsules using a capsule filling machine (SF-40N, Sejong Pharmatech Co., Ltd., South Korea), thereby preparing capsules.

### Example 44: Preparation of atorvastatin-aliskiren press-coated tablets

### 1) Preparation of atorvastatin prior-release granules

For Example 44, according to the contents given in Table 5, atorvastatin, microcrystalline cellulose, corn starch, and precipitated calcium carbonate were sieved through a No. 35 sieve and mixed in a high-speed mixer (Lab. Pharma Mixer P, Diosna, Germany). Meanwhile, hydroxypropylcellulose was dissolved in water to prepare a binding solution. The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the dried material was sieved again through a No. 20 sieve (KYK-60, KoreaMedi Co., Ltd., South Korea). Sodium starch glycolate was added to the sieved material, followed by mixing in a double cone mixer (Dasan Pharmatech, South Korea). Magnesium stearate was added to the mixture, followed by final mixing.

### 2) Preparation of aliskiren delayed-release coated tablets

For Example 44, according to the contents given in Table 5, aliskiren, D-mannitol, and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed granulator. Meanwhile, hydroxypropylmethylcellulose and citric acid were dissolved in water to prepare a binding solution which was then sprayed thereon to form granules, followed by drying. Then, powdered Carbomer 71 G was added to the granules, and magnesium stearate was added thereto, followed by final mixing in a double cone mixer (Dasan Pharmatech, South Korea).

The final mixture was compressed into tablets using a rotary tablet press (MRC-33, Sejong Pharmatech Co., Ltd., South Korea). Then, a hydroxypropylmethylcellulose phthalate solution (5% w/w) was coated thereon to prepare aliskiren delayed-release coated tablets.

### 3) Compression and coating

Using Press-coated tablet press (RUD-1: Kilian, Germany), press-coated tablets were prepared including the coated aliskiren tablets as an inner core and the atorvastatin granules as an outer layer. Meanwhile, hydroxypropylmethylcellulose 2910, hydroxypropylcellulose, titanium oxide, and talc were dissolved and dispersed in an 8:2 mixed solution of ethanol and purified water to prepare a coating solution (20%w/w). The compressed press-coated tablets were coated with the coating solution in a Hi-coater (SFC-30N, Sejong Pharmatech Co., Ltd., South Korea) to form a film-coated layer, thereby preparing press-coated tablets.

### Example 45: Atorvastatin-aliskiren blister package kits

### 1) Preparation of atorvastatin prior-release granules

According to the compositions and contents of Table 5, prior-release granules were prepared in the same manner as in 1) Preparation of atorvastatin prior-release granules in Example 38.

### 2) Preparation of aliskiren delayed-release granules

According to the compositions and contents of Table 5, delayed-release granules were prepared in the same manner as in 2) Preparation of aliskiren delayed-release granules in Example 38.

### 3) Compression and packaging

The final products of Processes 1) and 2) were compressed into tablets using a rotary tablet press (MRC-33, Sejong Pharmatech Co., Ltd., South Korea), and were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

### Example 46: Aliskiren-atorvastatin two-phase matrix tablets

According to the compositions of Table 6 below, two-phase matrix tablets were prepared in the same manner as in the preparation of two-phase matrix tablets in Example 30, except that a pharmacologically active ingredient of the prior-release compartment was aliskiren and an pharmacologically active ingredient of the delayed-release compartment was atorvastatin.

### 1) Preparation of aliskiren prior-release granules

According to the compositions and contents of Example 46 given in Table 6 below, prior-release granules were prepared in the same manner as in 1) Preparation of atorvastatin prior-release granules in Example 30, except that precipitated calcium carbonate was not used and the pharmacologically active ingredient was aliskiren.

### 2) Preparation of atorvastatin delayed-release granules

According to the compositions and contents of Example 46 given in Table 6, delayed-release granules were prepared in the same manner as in 2) Preparation of aliskiren delayed-release granules in Example 30, except that precipitated calcium carbonate was added and the pharmacologically active ingredient was atorvastatin.

### 3) Compression and packaging

The final products of Processes 1) and 2) were first mixed in a double cone mixer (Dasan Pharmatech, South Korea). To the first mixture was added sodium starch glycolate, followed by secondary mixing in a double cone mixer. Magnesium stearate was added to the secondary mixture, followed by final mixing.

Meanwhile, hydroxymethylcellulose 2910, hydroxypropylcellulose, titanium oxide, and talc were dissolved and dispersed in an 8:2 mixed solution of ethanol and purified water to prepare a coating solution (20%w/w). The compressed tablets were coated with the coating solution in a Hi-coater (SFC-30N, Sejong Pharmatech Co., Ltd., South Korea) to form a film-coated layer, thereby preparing a two-phase matrix preparation.

### Example 47: Preparation of aliskiren-atorvastatin capsules (tablets-tablets)

According to the compositions of Table 6 below, a preparation in the form of a capsule (tablet-tablet) was prepared in the same manner as in the preparation of capsules (tablets-tablets) in Example 42, except that an pharmacologically active ingredient of the prior-release compartment was aliskiren, and an pharmacologically active ingredient of the delayed-release compartment was atorvastatin.

### 1) Preparation of aliskiren prior-release tablets

According to the compositions and contents of Example 18 given in Table 6 below, prior-release tablets were prepared in the same manner as in 1) Preparation of atorvastatin prior-release tablets in Example 42, except that meglumine was not added and the pharmacologically active ingredient was aliskiren.

### 2) Preparation of atorvastatin delayed-release coated tablets

According to the compositions and contents of Example 47 given in Table 6 below, coated tablets were prepared in the same manner as in 2) Preparation of aliskiren delayed-release coated tablets in Example 42, except that a solution of hydroxypropylcellulose and meglumine in water was used as a binding solution, and the pharmacologically active ingredient was atorvastatin.

### 3) Capsule filling

The final products of Processes 1) and 2 were filled in capsules using a capsule filling machine (SF-40N, Sejong Pharmatech Co., Ltd., South Korea), thereby preparing capsules.

### Example 48: Aliskiren-atorvastatin multi-layered tablets

According to the compositions of Table 6 below, a preparation in the form of a multi-layered tablet was prepared in the same manner as in the preparation of multi-layered tablets in Example 32, except that an pharmacologically active ingredient of the prior-release compartment was aliskiren, and an pharmacologically active ingredient of the delayed-release compartment was atorvastatin.

### 1) Preparation of aliskiren prior-release granules

According to the compositions and contents of Example 48 given in Table 6 below, aliskiren prior-release granules were prepared in the same manner as in 1) Preparation of atorvastatin prior-release granules in Example 32, except that precipitated calcium carbonate was not used and the pharmacologically active ingredient was aliskiren.

### 2) Preparation of atorvastatin delayed-release granules

According to the compositions and contents of Example 48 given in Table 6, atorvastatin delayed-release granules were prepared in the same manner as in 2) Preparation of aliskiren delayed-release granules in Example 32, except that precipitated calcium carbonate was added and the pharmacologically active ingredient was atorvastatin.

### 3) Compression and coating

The product of Process 1) was placed in a first powder feeder, and the product of Process 2) was placed in a second powder feeder, followed by compression using a multi-layered tablet press (MRC-37T, Sejong Pharmatech Co., Ltd., South Korea). Meanwhile, hydroxymethylcellulose 2910, hydroxypropylcellulose, titanium oxide, and talc were dissolved and dispersed in an 8:2 mixed solution of ethanol and purified water to prepare a coating solution (20%w/w). The compressed tablets were coated with the coating solution in a Hi-coater (SFC-30N, Sejong Pharmatech Co., Ltd., South Korea) to form a film-coated layer, thereby preparing a preparation in the form of a multi-layered tablet.

### Example 49: Aliskiren-atorvastatin capsules (granules-granules)

According to the compositions of Table 6 below, capsules (granules-granules) were prepared in the same manner as in the preparation of capsules (granules-granules) in Example 38, except that an pharmacologically active ingredient of the prior-release compartment was aliskiren, and an pharmacologically active ingredient of the delayed-release compartment was atorvastatin.

### 1) Preparation of aliskiren prior-release granules

According to the compositions and contents of Example 49 given in Table 6 below, aliskiren prior-release granules were prepared in the same manner as in 1) Preparation of atorvastatin prior-release granules in Example 38, except that meglumine was not added and the pharmacologically active ingredient was aliskiren.

### 2) Preparation of atorvastatin delayed-release granules

According to the compositions and contents of Example 49 given in Table 6 below, delayed-release granules were prepared in the same manner as in 2) Preparation of aliskiren delayed-release granules in Example 38, except that a solution of hydroxypropylcellulose and meglumine in water was used as a binding solution, and the pharmacologically active ingredient was atorvastatin.

### 3) Capsule filling

The final products of Processes 1) and 2 were filled in capsules using a capsule filling machine (SF-40N, Sejong Pharmatech Co., Ltd., South Korea), thereby preparing a controlled-release preparation in the form of a capsule.

### Example 50: Aliskiren-atorvastatin capsules (granules-tablets)

According to the compositions of Table 6 below, a controlled-release preparation in the form of a capsule (granule-tablet) was prepared in the same manner as in the preparation of capsules (granules-tablets) in Example 40, except that a pharmacologically active ingredient of the prior-release layer was aliskiren, and an pharmacologically active ingredient of the delayed-release compartment was atorvastatin.

### 1) Preparation of aliskiren prior-release granules

According to the compositions and contents of Example 50 given in Table 6 below, aliskiren prior-release granules were prepared in the same manner as in Process 1) of Example 40, except that aliskiren instead of atorvastatin, and D-mannitol, microcrystalline cellulose and sodium starch glycolate were sieved through a No. 35 sieve and mixed in a high-speed mixer.

### 2) Preparation of atorvastatin delayed-release tablets

According to the compositions and contents of Example 50 given in Table 6, atorvastatin delayed-release granules were prepared in the same manner as in Process 2) of Example 40, except that atorvastatin instead of aliskiren, and D-mannitol, microcrystalline cellulose and precipitated calcium carbonate were sieved through a No. 35 sieve and mixed in a high-speed mixer.

### 3) Capsule filling

The final compositions of Processes 1) and 2) were filled in capsules using a capsule filling machine (SF-40N, Sejong Pharmatech Co., Ltd., South Korea), thereby preparing a controlled-release preparation in the form of a capsule.

**[Table 1]**

| Ingredients | | Content/unit preparation (mg) | | | | | | | | |
|---|---|---|---|---|---|---|---|---|---|---|
| | | Example No. | | | | | | | | |
| | | 1 | 2 | 3 | 4 | 5 | 6 | 7 | 8 | 9 |
| Delayed- release compartment | Aliskiren hemi-fumarate | 167.8 | 331.5 | 167.8 | | | | | | |
| | Atorvastatin calcium | | | | 21.7 | | | 21.7 | 21.7 | |
| | Simvastatin | | | | | 20 | | | | 20 |
| | Fluvastatin sodium | | | | | | 42.2 | | | |
| | Microcrystalline cellulose | 50 | 137 | 123 | 50 | 137 | 123 | 52 | 137 | 123 |
| | Crospovidone¹⁾ | 100 | | | 100 | | | 100 | | |
| | Polyvinylpyrrolidone | 18 | 18 | 18 | 18 | 18 | 18 | 18 | 18 | 18 |
| | Kollicoat SR30D²⁾ | | | 48 | | | 24 | | | 48 |
| | Hydroxypropylmethylcellulose | 4 | 8 | | 4 | 8 | | 4 | 8 | |
| | Hydroxypropylmethylcellulose phthalate | | 12 | | | 12 | | | 12 | |
| | Cellulose acetate (acetyl group 32%) | 40 | | | 40 | | | 40 | | |
| | Cellulose acetate (acetyl group 39.8%) | 40 | | | 40 | | | 40 | | |
| | Sodium chloride | 50 | | | 50 | | | 50 | | |
| | Citric acid | | | | 6.5 | 2 | 6.5 | 2 | 2 | 2 |
| | Butylated hydroxyanisole | | | | 0.35 | 0.1 | 0.35 | 0.1 | 0.1 | 0.1 |
| | Magnesium stearate | | | | | | | 3 | 3 | 3 |
| Prior-release compartment | Aliskiren hemi-fumarate | | | | 331.5 | 167.8 | 167.8 | 167.8 | 167.8 | 167.8 |
| | Atorvastatin calcium | 21.7 | | | | | | | | |
| | Simvastatin | | 20 | | | | | | | |
| | Fluvastatin sodium | | | 42.2 | | | | | | |
| | Microcrystalline cellulose | 95 | 95 | 57 | 95 | 57 | 95 | 57 | 57 | 57 |
| | D-mannitol | | | 111.4 | | 115.6 | | 111.9 | 113.9 | 111.6 |
| | Lactose | | 272.6263.6 | | 264.9 | | 283.1 | | | |
| | Corn starch | 50 | 50 | | 50 | | 50 | | | |
| | Sodium starch glycolate | 15 | 15 | 2 | 15 | 2 | 15 | 2 | 2 | 2 |
| | Butylated hydroxyanisole | 0.35 | 0.35 | 0.1 | | | | | | |
| | Hydroxypropylcellulose | 10 | 10 | 5 | 10 | 5 | 10 | 5 | 5 | 5 |
| | Citric acid | 6.5 | 6.5 | 2 | | | | | | |
| | Magnesium stearate | 0 | 0 | 0 | 0 | 0 | 0 | 1.5 | 1.5 | 1.5 |
| Post-mixing | Magnesium stearate | 5.05 | 5.05 | 4.5 | 5.05 | 4.5 | 5.05 | | | |
| Coating layer | Hydroxypropylmethylcellulose 2910 | 9 | 8 | 5.5 | 8 | 5.5 | 8 | 7 | 5.5 | 5.5 |
| | Hydroxypropylcellulose | 9 | 8 | 5.5 | 8 | 5.5 | 8 | 7 | 5.5 | 5.5 |
| | Titanium oxide | 8 | 7 | 4.8 | 7 | 4.8 | 7 | 6 | 4.8 | 4.8 |
| | Talc | 5 | 5 | 3.2 | 5 | 3.2 | 5 | 4 | 3.2 | 3.2 |
| Total | | 977 | 1000 | 600 | 1130 | 568 | 868 | 700 | 568 | 578 |
| ¹⁾Crospovidone - Main ingredient: polyvinylpyrrolidone (BASF) | | | | | | | | | | |
| ²⁾Kollicoat SR30D - Main ingredient: polyvinyl acetate 30% suspension (BASF) | | | | | | | | | | |

**[Table 2]**

| Ingredients | | Content/unit preparation (mg) | | | | | | | | |
|---|---|---|---|---|---|---|---|---|---|---|
| | | Example No. | | | | | | | | |
| | | 10 | 11 | 12 | 13 | 14 | 15 | 16 | 17 | 18 |
| Delayed-release compartment | Aliskiren hemi-fumarate | 167.8 | 167.8 | 167.8 | 167.8 | 167.8 | 167.8 | 167.8 | 167.8 | 167.8 |
| | Microcrystalline cellulose | 50 | 137 | 120.2 | 112.2 | 92.2 | 100.2 | 87.2 | 83.2 | 69.2 |
| | Pregelatinized starch | | | 40 | 40 | 40 | 40 | 40 | 40 | 40 |
| | Polyvinylpyrrolidone | 18 | 18 | 18 | 18 | 18 | 18 | 18 | 18 | 18 |
| | Colloidal silicon dioxide | | | 4 | 4 | 4 | 4 | 4 | 4 | 4 |
| | Crospovidone¹⁾ | 100 | | | | 15 | | 20 | 24 | 38 |
| | Hydroxypropylmethylcellulose | 4 | 8 | | | | | | | |
| | Hydroxypropylmethylcellulose phthalate | | 12 | | | | | | | |
| | Cellulose acetate (acetyl group 32%) | 40 | | | | | | | | |
| | Cellulose acetate (acetyl group 39.8%) | 40 | | | | | | | | |
| | Ethylcellulose | | | 12 | 20 | 25 | 32 | 25 | 25 | 25 |
| | Methacrylic acid copolymer type C²⁾ | | | 32 | 32 | 32 | 32 | 32 | 32 | 32 |
| | Sodium chloride | 50 | | | | | | | | |
| | Magnesium stearate | 3 | 3 | 6 | 6 | 6 | 6 | 6 | 6 | 6 |
| Prior-release compartment | Atorvastatin calcium | | | 21.7 | 21.7 | 21.7 | 21.7 | 21.7 | 21.7 | 21.7 |
| | Simvastatin | 20 | | | | | | | | |
| | Rosuvastatin | | 40 | | | | | | | |
| | Microcrystalline cellulose | 57 | 57 | 95 | 95 | 95 | 95 | 95 | 95 | 95 |
| | D-mannitol | 112.6 | 112.6 | | | | | | | |
| | Lactose | | | 196.4 | 196.4 | 196.4 | 196.4 | 196.4 | 196.4 | 196.4 |
| | Corn starch | | | 50 | 50 | 50 | 50 | 50 | 50 | 50 |
| | Sodium starch glycolate | 2 | 2 | 15 | 15 | 15 | 15 | 15 | 15 | 15 |
| | Butylated hydroxyanisole | 0.1 | 0.1 | 0.35 | 0.35 | 0.35 | 0.35 | 0.35 | 0.35 | 0.35 |
| | Hydroxypropylcellulose | 5 | 5 | 10 | 10 | 10 | 10 | 10 | 10 | 10 |
| | Citric acid | 2 | 2 | 6.5 | 6.5 | 6.5 | 6.5 | 6.5 | 6.5 | 6.5 |
| | Magnesium stearate | 1.5 | 1.5 | 5.05 | 5.05 | 5.05 | 5.05 | 5.05 | 5.05 | 5.05 |
| Coating layer | Hydroxypropylmethylcellulose 2910 | 6.5 | 5.5 | 23 | 23 | 23 | 23 | 23 | 25 | 25 |
| | Hydroxypropylcellulose | 6.5 | 5.5 | | | | | | | |
| | Titanium oxide | 5.5 | 4.8 | 3 | 3 | 3 | 3 | 3 | 3.5 | 3.5 |
| | Talc | 3.5 | 3.2 | 2 | 2 | 2 | 2 | 2 | 2.5 | 2.5 |
| Total | | 695 | 585 | 828 | 828 | 828 | 828 | 828 | 831 | 831 |
| ¹⁾Crospovidone - Main ingredient: polyvinylpyrrolidone (BASF) | | | | | | | | | | |
| ²⁾Methacrylic acid copolymer type C - trade name: Eudragit L100-55 (Degussa) | | | | | | | | | | |

**[Table 3]**

| Ingredients | | Content/unit preparation (mg) | | | | | | | | |
|---|---|---|---|---|---|---|---|---|---|---|
| | | Example No. | | | | | | | | |
| | | 19 | 20 | 21 | 22 | 23 | 24 | 25 | 26 | 27 |
| Delayed- release compartment | Aliskiren hemi-fumarate | 167.8 | | 167.8 | | 167.8 | | 167.8 | | |
| | Simvastatin | | 20 | | | | | | | 21.7 |
| | Fluvastatin sodium | | | | | | | | 42.2 | |
| | Pitavastatin calcium | | | | 2 | | | | | |
| | Rosuvastatin | | | | | | 40 | | | |
| | Microcrystalline cellulose | | | | | 123 | 123 | 137 | 137 | 14 |
| | Sugar seed | 35 | 35 | 35 | 35 | | | | | |
| | Kollicoat SR30D¹⁾ | | | | | 24 | 24 | | | |
| | Hydroxypropylmethylcellulose | 5 | 5 | 5 | 5 | | | 8 | 8 | 0.8 |
| | Pregelatinized starch | | | | | | | | | 10 |
| | Polyvinylpyrrolidone | | | | | | | | | 4.5 |
| | Hydroxypropylmethylcellulose phthalate | 45 | 45 | 45 | 45 | | | 12 | 12 | |
| | Methacrylic acid copolymer (type C2) | | | | | | | | | 8 |
| | Citric acid | | 2 | | 2 | | 2 | | 2 | 2 |
| | Magnesium stearate | | | | | | | | | 1.5 |
| Prior-release compartment | Aliskiren hemi-fumarate | | 167.8 | | 167.8 | | 167.8 | | 167.8 | 167.8 |
| | Atorvastatin calcium | | | | | | | 21.7 | | |
| | Pravastatin sodium | | | 20 | | | | | | |
| | Pitavastatin calcium | 2 | | | | | | | | |
| | Rosuvastatin | | | | | | 0 | | | |
| | Lovastatin | | | | | 20 | | | | |
| | Microcrystalline cellulose | 57 | 57 | 57 | 57 | 57 | 57 | 57 | 57 | 57 |
| | D-mannitol | 124.6 | 106.7 | 106.6 | 124.7 | 104.6 | 124.7 | 102.9 | 112.5 | 119.5 |
| | Sodium starch glycolate | | 0 | 2 | 2 | 0 | 0 | 2 | 2 | 2 |
| | Butylated hydroxyanisole | 0.1 | | 0.1 | | 0.1 | | 0.1 | | |
| | Hydroxypropylcellulose | 5 | 5 | 5 | 5 | 5 | 5 | 5 | 5 | 5 |
| | Citric acid | 2 | | 2 | | 2 | | 2 | | |
| | Magnesium stearate | | 0 | 4.5 | 4.5 | 0 | 0 | 4.5 | 4.5 | 4.5 |
| st-mixing | Sodium starch glycolate | 2 | 2 | | | 2 | 2 | | | |
| | Magnesium stearate | 4.5 | 4.5 | | | 4.5 | 4.5 | | | |
| Coating layer | Hydroxypropylmethylcellulose 2910 | | | | | | | | | 25.6 |
| | Hydroxypropylcellulose | | | | | | | | | |
| | Titanium oxide | | | | | | | | | 3.7 |
| | Talc | | | | | | | | | 2.4 |
| Total | | 450 | 450 | 450 | 450 | 510 | 550 | 520 | 550 | 450 |
| ¹⁾Kollicoat SR30D - Main ingredient: polyvinyl acetate 30% suspension (BASF) | | | | | | | | | | |

**[Table 4]**

| Ingredients | | Content/unit preparation (mg) | | | | | | | |
|---|---|---|---|---|---|---|---|---|---|
| | | Example No. | | | | | | | |
| | | 30 | 31 | 32 | 33 | 34 | 35 | 36 | 37 |
| Prior-release compartment | Atorvastatin calcium anhydride | 10.35 | 0 | 10.35 | 0 | 10.35 | 0 | 10.35 | 0 |
| | Atorvastatin calcium trihydrate | 0 | 10.85 | 0 | 0 | 0 | 10.85 | 0 | 0 |
| | Atorvastatin strontium pentahydrate | 0 | 0 | 0 | 11.6 | 0 | 0 | 0 | 11.6 |
| | Microcrystalline cellulose | 24.15 | 23.65 | 24.15 | 22.9 | 26.65 | 25.15 | 24.15 | 22.9 |
| | Corn starch | 10 | 10 | 15 | 15 | 15 | 15 | 15 | 15 |
| | Sodium starch glycolate | 0 | 0 | 2 | 2 | 0 | 0 | 2 | 2 |
| | Hydroxypropylcellulose | 5 | 5 | 5 | 5 | 5 | 5 | 5 | 5 |
| | Meglumine | 0 | 0 | 0 | 0 | 2 | 2 | 0 | 0 |
| | Precipitated calcium carbonate | 2 | 2 | 2 | 2 | 0 | 0 | 2 | 2 |
| | Magnesium stearate | 0 | 0 | 1.5 | 1.5 | 0 | 0 | 1.5 | 1.5 |
| Delayed-release compartment | Aliskiren hemi-fumarate | 167.8 | 167.8 | 167.8 | 167.8 | 167.8 | 167.8 | 167.8 | 167.8 |
| | D-mannitol | 127 | 127 | 127 | 127 | 117.2 | 117.2 | 118.7 | 118.7 |
| | Lactose | 15 | 15 | 15 | 15 | 0 | 0 | 0 | 0 |
| | Sugar sphere | 0 | 0 | 0 | 0 | 70 | 70 | 70 | 70 |
| | Microcrystalline cellulose | 68.2 | 68.2 | 71.7 | 71.7 | 25.5 | 25.5 | 24 | 24 |
| | Eudragit RS PO¹⁾ | 20 | 20 | 0 | 0 | 0 | 0 | 0 | 0 |
| | Hydroxypropylmethylcellulose | 2 | 2 | 7 | 7 | 7 | 7 | 7 | 7 |
| | Hydroxypropylmethylcellulose phthalate | 0 | 0 | 10 | 10 | 10 | 10 | 10 | 10 |
| | Citric acid | 0 | 0 | 0 | 0 | 1 | 1 | 1 | 1 |
| | Magnesium stearate | 0 | 0 | 1.5 | 1.5 | 0 | 0 | 1.5 | 1.5 |
| Post-mixing | Sodium starch glycolate | 7 | 7 | 0 | 0 | 2 | 2 | 0 | 0 |
| | Magnesium stearate | 1.5 | 1.5 | 0 | 0 | 1.5 | 1.5 | 0 | 0 |
| Coating layer | Hydroxymethylcellulose 2910 | 6.6 | 6.6 | 6.6 | 6.6 | 0 | 0 | 0 | 0 |
| | Hydroxypropylcellulose | 6.6 | 6.6 | 6.6 | 6.6 | 0 | 0 | 0 | 0 |
| | Titanium oxide | 5.8 | 5.8 | 5.8 | 5.8 | 0 | 0 | 0 | 0 |
| | Talc | 4 | 4 | 4 | 4 | 0 | 0 | 0 | 0 |
| Total | | 483 | 483 | 483 | 483 | 461 | 460 | 460 | 460 |
| ¹⁾Eudragit RS PO - Main ingredient: polyammoniomethacrylate copolymer (Degussa) | | | | | | | | | |

**[Table 5]**

| Ingredients | | Content/unit preparation (mg) | | | | | | | |
|---|---|---|---|---|---|---|---|---|---|
| | | Example No. | | | | | | | |
| | | 38 | 39 | 40 | 41 | 42 | 43 | 44 | 45 |
| Prior-release compartment | Atorvastatin calcium anhydride | 10.35 | 0 | 10.35 0 | | 10.35 0 | | 10.35 | 10.35 |
| | Atorvastatin calcium trihydrate | 0 | 10.85 | 0 | 0 | 0 | 10.85 | 0 | 0 |
| | Atorvastatin strontium pentahydrate | 0 | 0 | 0 | 11.6 | 0 | 0 | 0 | 0 |
| | Microcrystalline cellulose | 20 | 20 | 20 | 20 | 20 | 20 | 20 | 20 |
| | D-mannitol | 20.65 | 20.15 | 20.65 | 19.4 | 0 | 0 | 0 | 20.65 |
| | Lactose | 0 | 0 | 0 | 0 | 0 | 0 | 0 | 0 |
| | Corn starch | 0 | 0 | 0 | 0 | 19.15 | 18.65 0 | 19.15 | 0 |
| | Sodium starch glycolate | 0 | 0 | 0 | 0 | 2 | 2 | 2 | 0 |
| | Hydroxypropylcellulose | 5 | 5 | 5 | 5 | 5 | 5 | 5 | 5 |
| | Meglumine | 2 | 2 | 0 | 0 | 2 | 2 | 0 | 2 |
| | Precipitated calcium carbonate | 0 | 0 | 2 | 2 | 0 | 0 | 2 | 0 |
| | Magnesium stearate | 0 | 0 | 0 | 0 | 1.5 | 1.5 | 1.5 | 0 |
| Delayed-release compartment | Aliskiren hemi-fumarate | 167.8 | 167.8 | 167.8 | 167.8 | 167.8 | 167.8 | 167.8 | 167.8 |
| | D-mannitol | 185.1 | 185.1 | 187.1 | 187.1 | 187.1 | 187.1 | 187.1 | 185.1 |
| | Sugar sphere | 0 | 0 | 0 | 0 | 0 | 0 | 0 | 0 |
| | Microcrystalline cellulose | 28.6 | 28.6 | 26.6 | 26.6 | 23.6 | 23.6 | 23.6 | 30.6 |
| | Kollicoat SR30D¹⁾ | 15 | 15 | 0 | 0 | 0 | 0 | 0 | 15 |
| | Carbomer 71 G²⁾ | 0 | 0 | 10 | 10 | 10 | 10 | 10 | 0 |
| | Hydroxypropylmethylcellulose | 2 | 2 | 2 | 2 | 2 | 2 | 2 | 2 |
| | Hydroxypropylmethylcellulose phthalate | 0 | 0 | 7 | 7 | 7 | 7 | 7 | 0 |
| | Citric acid | 0 | 0 | 0 | 0 | 1 | 1 | 1 | 0 |
| | Magnesium stearate | 0 | 0 | 1.5 | 1.5 | 1.5 | 1.5 | 1.5 | 1.5 |
| Post-mixing | Sodium starch glycolate | 2 | 2 | 0 | 0 | 0 | 0 | 0 | 0 |
| | Magnesium stearate | 1.5 | 1.5 | 0 | 0 | 0 | 0 | 0 | 0 |
| Coating layer | Hydroxymethylcellulose 2910 | 0 | 0 | 0 | 0 | 0 | 0 | 6.6 | 0 |
| | Hydroxypropylcellulose | 0 | 0 | 0 | 0 | 0 | 0 | 6.6 | 0 |
| | Titanium oxide | 0 | 0 | 0 | 0 | 0 | 0 | 5.8 | 0 |
| | Talc | 0 | 0 | 0 | 0 | 0 | 0 | 4 | 0 |
| Total | | 460 | 460 | 460 | 460 | 460 | 460 | 483 | 460 |
| ¹⁾Kollicoat SR30D - Main ingredient: polyvinyl acetate 30% suspension (BASF) | | | | | | | | | |
| ²⁾Carbomer 71G - Main ingredient: Carboxyvinyl polymer (Lubrizol) | | | | | | | | | |

**[Table 6]**

| Ingredients | | Content/unit preparation (mg) | | | | |
|---|---|---|---|---|---|---|
| | | Example No. | | | | |
| | | 46 | 47 | 48 | 49 | 50 |
| Prior-release compartment | Aliskiren hemi-fumarate | 167.8 | 167.8 | 167.8 | 167.8 | 167.8 |
| | Microcrystalline cellulose | 105.2 | 103.55 | 108.7 | 102.55 | 102.55 |
| | D-mannitol | 0 | 0 | 0 | 22.65 | 22.65 |
| | Lactose | 0 | 0 | 0 | 0 | 0 |
| | Corn starch | 10 | 20.15 | 15 | 0 | 0 |
| | Sodium starch glycolate | 0 | 2 | 2 | 0 | 2 |
| | Hydroxypropylcellulose | 5 | 5 | 5 | 5 | 5 |
| | Citric acid | 0 | 0 | 0 | 0 | 0 |
| | Magnesium stearate | 0 | 1.5 | 1.5 | 0 | 0 |
| Delayed-release compartment | Atorvastatin calcium anhydride | 10.35 | 10.35 | 10.35 | 10.35 | 10.35 |
| | D-mannitol | 127 | 142 | 127 | 183.1 | 185.1 |
| | Lactose | 15 | 0 | 15 | 0 | 0 |
| | Sugar sphere | 0 | 0 | 0 | 0 | 0 |
| | Microcrystalline cellulose | 104.15 | 102.15 | 104.15 | 86.05 | 82.05 |
| | Eudragit RS PO¹⁾ | 20 | 0 | 0 | 0 | 0 |
| | Kollicoat SR30D²⁾ | 0 | 0 | 0 | 15 | 0 |
| | Carbomer 71G³⁾ | 0 | 10 | 0 | 0 | 10 |
| | Hydroxypropylmethylcellulose | 2 | 2 | 7 | 2 | 2 |
| | Hydroxypropylmethylcellulose phthalate | 0 | 7 | 10 | 0 | 7 |
| | Meglumine | 0 | 2 | 0 | 2 | 0 |
| | Precipitated calcium carbonate | 2 | 0 | 2 | 0 | 2 |
| | Magnesium stearate | 0 | 1.5 | 1.5 | 0 | 1.5 |
| Post-mixing | Sodium starch glycolate | 7 | 0 | 0 | 2 | 0 |
| | Magnesium stearate | 1.5 | 0 | 0 | 1.5 | 0 |
| Coating layer | Hydroxymethylcellulose 2910 | 6.6 | 6.6 | 6.6 | 0 | 0 |
| | Hydroxypropylcellulose | 6.6 | 6.6 | 6.6 | 0 | 0 |
| | Titanium oxide | 5.8 | 5.8 | 5.8 | 0 | 0 |
| | Talc | 4 | 4 | 4 | 0 | 0 |
| Total | | 600 | 600 | 600 | 600 | 600 |
| ¹⁾Eudragit RS PO - Main ingredient: polyammoniomethacrylate copolymer (Degussa) | | | | | | |
| ²⁾Kollicoat SR30D - Main ingredient: polyvinyl acetate 30% suspension (BASF) | | | | | | |
| ³⁾Carbomer 71G - Main ingredient: Carboxyvinyl polymer (Lubrizol) | | | | | | |

### Experimental Example 1: Comparative dissolution profile test

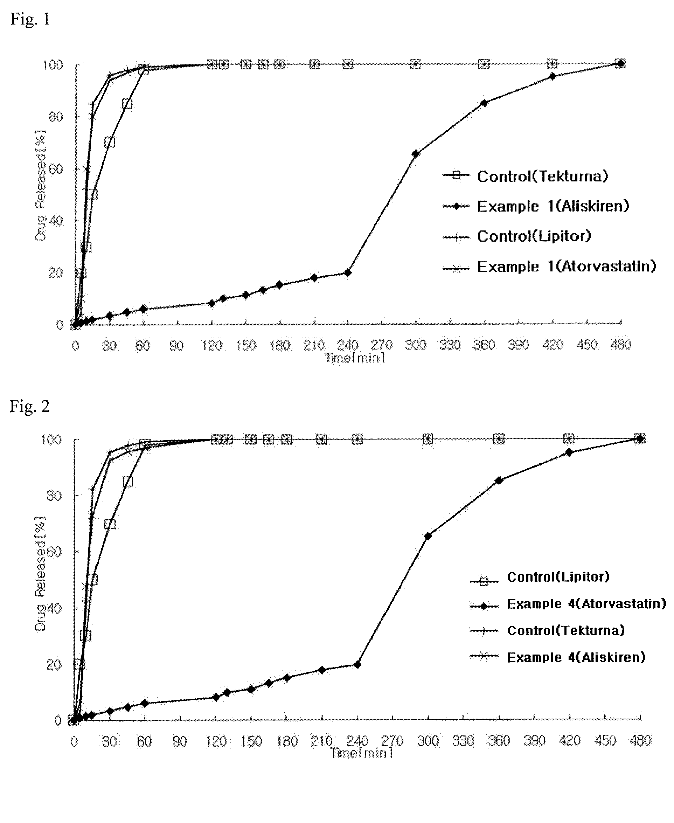

A comparative dissolution profile test was performed using aliskiren/atorvastatin two-phase matrix tablets prepared in Example 1 and control drugs (Lipitor: atorvastatin single drug, Tekturna: aliskiren single drug). The dissolution profile test of the atorvastatin ingredient was performed based on the general dissolution test method described in the Korean Pharmacopoeia (8^{th} revision), and the dissolution profile test of the aliskiren ingredient was performed for a total of 480 minutes, in which the dissolution medium was changed from a simulated gastric juice to a dissolution medium of the test method 120 minutes after the start of the test. Details of the dissolution profile test of each ingredient are as follows. The results obtained are shown in FIG. 1.

FIG. 1 is a graph showing the comparative dissolution profiles of an aliskiren-atorvastatin preparation prepared in Example 1, and the aliskiren and atorvastatin ingredients of single drugs, Tekturna and Lipitor, as control drugs. In FIG. 1, the x-axis represents the time series, and the y-axis represents the dissolution rate (%).

As can be seen in FIG. 1, when the dissolution profile test was performed under the following conditions, the atorvastatin ingredient of the two-phase matrix tablet of the present invention showed a dissolution profile substantially equal to that of the control drug Lipitor, but the aliskiren ingredient showed a very slow dissolution rate as compared to that of the control drug Tekturna. In the dissolution profile test results for the aliskiren ingredient, the dissolution rates of the aliskiren ingredient up to 120 minutes corresponding to the simulated gastric juice zone were all less than 10% in the aliskiren/atorvastatin two-phase matrix tablets of the present invention, but the control drugs showed the completion of release. The dissolution rate of the aliskiren ingredient in the subsequent dissolution medium of the test method zone was 100% in the control formulation, but was about 20% up to a total of 240 minutes in the aliskiren/atorvastatin two-phase combination controlled-release tablets of the present invention, which was far lower than that of the control drugs.

As described above, the initial release of aliskiren in the aliskiren/atorvastatin two-phase matrix tablets of the present invention is much slower than atorvastatin, unlike dissolution profiles obtained when the aliskiren single drug and the atorvastatin single drug, as the control drugs, are administered simultaneously. Thus, in the case of the inventive tablets, the time for metabolism-related enzyme cytochrome P450 to be regenerated after atorvastatin is metabolized first in the liver can be sufficiently ensured.

Test Method for atorvastatin calcium: Based on the general dissolution test method described in the Korean Pharmacopoeia (8^{th} revision)

Test method: Paddle method, 50 rpm

Dissolution medium: pH 7.0 buffer solution (composition = 0.01 M sodium dihydrogen phosphate solution containing sodium lauryl sulfate 2% wt/wt as surfactant), 900 mL

Analysis method: High performance liquid chromatography

Test Method for aliskiren hemi-fumarate: Based on the general dissolution test method described in the Korean Pharmacopoeia (8^{th} revision)

Test method: Paddle method, 50 rpm

Dissolution medium: 0.01 M hydrochloric acid solution 750 mL (simulated gastric juice), pH 6.8 phosphate buffer 1,000 mL (simulated intestinal juice)

Analysis method: UV Vis spectrophotometry

### Experimental Example 2: Comparative dissolution profiles test

A comparative dissolution profile test was performed using aliskiren/atorvastatin two-phase matrix tablets prepared in Example 4 and control drugs (Lipitor: atorvastatin single drug, Tekturna: aliskiren single drug). The dissolution profile test of the atorvastatin ingredient was performed based on the United States Pharmacopoeia (USP30), and the dissolution profile test of the aliskiren ingredient was performed based on the general dissolution test method described in the Korean Pharmacopoeia (8^{th} revision) for a total of 480 minutes, in which the dissolution medium was changed from a simulated gastric juice to a dissolution medium of the test method 120 minutes after the start of the test. The results obtained are shown in FIG. 2. Details of the dissolution profile test and analysis of aliskiren and atorvastatin ingredients are the same as those of Experimental Example 1. FIG. 2 is a graph showing the comparative dissolution profiles of an aliskiren-atorvastatin preparation prepared in Example 4, and the aliskiren and atorvastatin ingredients of single drugs, Tekturna and Lipitor, as control drugs. In FIG. 2, the x-axis represents the time series, and the y-axis represents the dissolution rate (%).

As can be seen in FIG. 2, when the dissolution profile test was performed under the following conditions, the aliskiren ingredient of the two-phase matrix tablet of the present invention showed a dissolution profile substantially equal to that of the control drug Tekturna, but the atorvastatin ingredient showed a very slow dissolution rate as compared to that of the control drug Lipitor. In the dissolution profile test results for the atorvastatin ingredient, the dissolution rates of the atorvastatin ingredient up to 120 minutes corresponding to the simulated gastric juice zone were all less than 10% in the aliskiren/atorvastatin two-phase matrix tablets of the present invention, but the control drugs showed the completion of release. The dissolution rate of the atorvastatin ingredient in the subsequent dissolution medium of the test method zone was 100% in the control formulation, but was about 20% up to a total of 240 minutes in the aliskiren/atorvastatin two-phase matrix tablets of the present invention, which was far lower than that of the control drugs.

As described above, the initial release of atorvastatin in the aliskiren/atorvastatin two-phase combination controlled-release tablets of the present invention is much slower than aliskiren, unlike dissolution profiles obtained when the aliskiren single drug and the atorvastatin single drug, as the control drugs, are administered simultaneously. Thus, in the case of the inventive tablets, the time for metabolism-related enzyme cytochrome P450 to be regenerated after aliskiren is metabolized first in the liver can be sufficiently ensured.

### Experimental Example 3: Comparative dissolution profile test

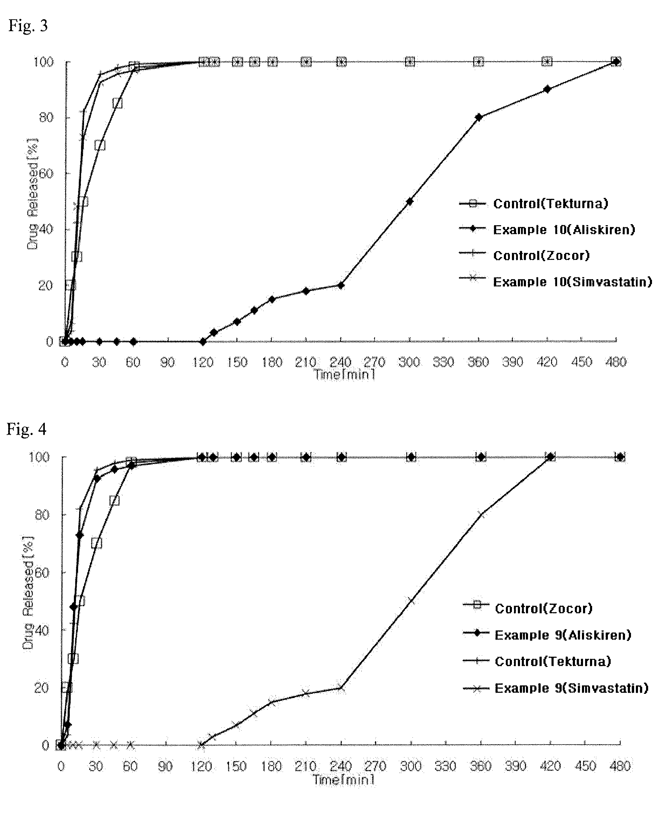

A comparative dissolution profile test was performed using aliskiren/simvastatin multi-layered tablets prepared in Example 10 and control drugs (Zocor: simvastatin single drug, Tekturna: aliskiren single drug). The dissolution profile test of the simvastatin ingredient was performed based on the general dissolution test method described in the Korean Pharmacopoeia (8^{th} revision), and the dissolution profile test of the aliskiren ingredient was performed for a total of 480 minutes, in which the dissolution medium was changed from a simulated gastric juice to a dissolution medium of the test method 120 minutes after the start of the test. Details of the dissolution profile test of the simvastatin ingredient are as follows. The results obtained are shown in FIG. 3. Details of the dissolution profile test and analysis of the aliskiren ingredient are the same as those of Experimental Example 1. FIG. 3 is a graph showing the comparative dissolution profiles of an aliskiren-simvastatin multi-layered tablet prepared in Example 10, and the aliskiren and simvastatin ingredients of single drugs, Tekturna and Zocor, as control drugs. In FIG. 3, the x-axis represents the time series, and the y-axis represents the dissolution rate (%).

As can be seen in FIG. 3, when the dissolution profile test was performed under the following conditions, the simvastatin ingredient of the multi-layered tablet of the present invention showed a dissolution profile substantially equal to that of the control drug Zocor, but the aliskiren (→renin inhibitor) ingredient showed a very slow dissolution rate as compared to that of the control drug Tekturna. In the dissolution profile test results for the aliskiren ingredient, the dissolution rates of the aliskiren ingredient up to 120 minutes corresponding to the simulated gastric juice zone were all less than 10% in the aliskiren/simvastatin two-phase controlled-release multi-layered tablets of the present invention, but the control drugs showed the completion of release. The dissolution rate of the aliskiren ingredient in the subsequent dissolution medium of the test method zone was 100% in the control formulation, but was about 20% up to a total of 240 minutes in the aliskiren/simvastatin two-phase controlled-release multi-layered tablets of the present invention, which was far lower than that of the control drugs.

As described above, the initial release of aliskiren in the aliskiren/simvastatin two-phase controlled-release multi-layered tablets of the present invention is much slower than simvastatin, unlike dissolution profiles obtained when the aliskiren single drug and the simvastatin single drug, as the control drugs, are administered simultaneously. Thus, in the case of the inventive tablets, the time for metabolism-related enzyme cytochrome P450 to be regenerated after simvastatin is metabolized first in the liver can be sufficiently ensured.

Test Method for simvastatin: Based on the "Simvastatin tablet" part in USP 30

Test method: Paddle method, 50 rpm

Dissolution medium: pH 7.0 buffer solution (composition = 0.01 M sodium dihydrogen phosphate solution containing sodium lauryl sulfate 0.5% wt/wt as surfactant), 900 mL

Analysis method: UV-Vis spectrophotometry

### Experimental Example 4: Comparative dissolution profile test

A comparative dissolution profile test was performed using aliskiren/simvastatin two-phase controlled-release multi-layered tablets prepared in Example 9 and control drugs (Zocor: simvastatin single drug, Tekturna: aliskiren single drug). The dissolution profile test of the aliskiren ingredient was performed based on the general dissolution test method described in the Korean Pharmacopoeia (8^{th} revision), and the dissolution profile test of the simvastatin ingredient was performed for a total of 480 minutes, in which the dissolution medium was changed from a simulated gastric juice to a dissolution medium of the test method 120 minutes after the start of the test. The results obtained are shown in FIG. 4. Details of the dissolution profile test of the aliskiren and simvastatin ingredients are the same as those of Experimental Example 3. FIG. 4 is a graph showing the comparative dissolution profiles of an aliskiren-simvastatin multi-layered tablet prepared in Example 9, and the aliskiren and simvastatin ingredients of single drugs, Tekturna and Zocor, as control drugs. In FIG. 4, the x-axis represents the time series, and the y-axis represents the dissolution rate (%).

As can be seen in FIG. 4, when the dissolution profile test was performed under the following conditions, the aliskiren ingredient of the multi-layered tablet of the present invention showed a dissolution profile substantially equal to that of the control drug Tekturna, but the simvastatin ingredient showed a very slow dissolution rate as compared to that of the control drug Zocor. In the dissolution profile test results for the simvastatin ingredient, the dissolution rates of the simvastatin ingredient up to 120 minutes corresponding to the simulated gastric juice zone were all less than 10% in the aliskiren/simvastatin multi-layered tablets of the present invention, but the control drugs showed the completion of release. The dissolution rate of the simvastatin ingredient in the subsequent dissolution medium of the test method zone was 100%, but was about 20% up to a total of 240 minutes in the aliskiren/simvastatin multi-layered tablets of the present invention, which was far lower than that of the control drugs.

As described above, the initial release of simvastatin in the aliskiren/simvastatin multi-layered tablets of the present invention is much slower than aliskiren, unlike dissolution profiles obtained when the aliskiren single drug and the simvastatin single drug, as the control drugs, are administered simultaneously. Thus, in the case of the inventive tablets, the time for metabolism-related enzyme cytochrome P450 to be regenerated after aliskiren is metabolized first in the liver can be sufficiently ensured.

### Experimental Example 5: Comparative dissolution profiles test

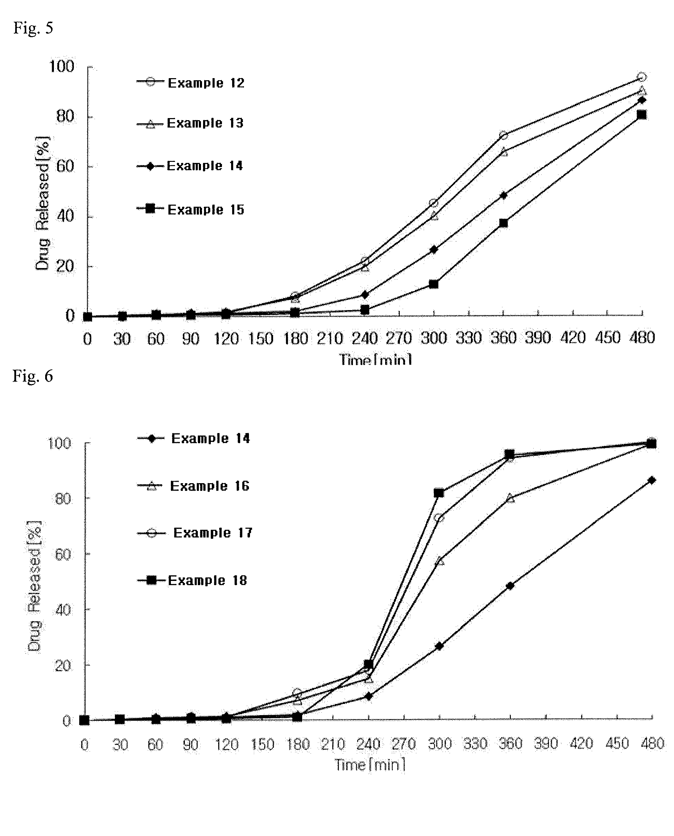

A comparative dissolution profile test was performed for Examples 12 to 15. Details of the dissolution profile test of each ingredient are the same as those of Experimental Example 1. The results obtained are shown in FIG. 5. FIG. 5 is a graph showing the dissolution profiles of Examples 12 to 15. In FIG. 5, the x-axis represents the time series, and the y-axis represents the dissolution rate (%).

As can be seen in FIG. 5, when the dissolution profile test was performed under the conditions of Experimental Example 1, the multi-layered tablet of the present invention showed a significant decrease in dissolution rate of the aliskiren ingredient in response to an increase in the amount of ethylcellulose to be used. When coated with ethylcellulose, Examples 12 to 15 exhibited the aliskiren dissolution rate of less than 20% up to a total of 240 minutes.

Therefore, the initial release of aliskiren in the aliskiren/atorvastatin multi-layered tablet of the present invention can be delayed up to the intended time by controlling the amount of ethylcellulose coated.

As described above, the initial release of aliskiren in the aliskiren/atorvastatin controlled-release multi-layered tablets of the present invention is much slower than atorvastatin, unlike dissolution profiles obtained when the aliskiren single drug and the atorvastatin single drug, as the control drugs, are administered simultaneously. Thus, in the case of the inventive tablets, the time for metabolism-related enzyme cytochrome P450 to be regenerated after atorvastatin is metabolized first in the liver can be sufficiently ensured.

### Experimental Example 6: Comparative dissolution profiles test

A comparative dissolution profile test was performed for Examples 14, and 16 to 18. Details of the dissolution profile test of each ingredient are the same as those of Experimental Example 1. The results obtained are shown in FIG. 6. FIG. 6 is a graph showing the dissolution profiles of Examples 14, and 16 to 18. In FIG. 6, the x-axis represents the time series, and the y-axis represents the dissolution rate (%).

As can be seen in FIG. 6, when the dissolution profile test was performed under the conditions of Experimental Example 1, the controlled-release multi-layered tablet of the present invention showed relatively rapid release of the aliskiren ingredient after an intended lag time when crospovidone is incorporated in the ethylcellulose-coated delayed-release layer. The dissolution rate of the aliskiren ingredient was less than 20% up to a total of 240 minutes and the aliskiren ingredient was rapidly released with an increase in the amount of crospovidone.

Therefore, the aliskiren/atorvastatin multi-layered tablet of the present invention can achieve rapid release of aliskiren after an intended lag time by controlling the content of crospovidone in the ethylcellulose-coated delayed-release layer.

As described above, the initial release of aliskiren in the aliskiren/atorvastatin multi-layered tablet of the present invention is much slower than atorvastatin, unlike dissolution profiles obtained when the aliskiren single drug and the atorvastatin single drug, as the control drugs, are administered simultaneously. Thus, in the case of the inventive tablets, the time for metabolism-related enzyme cytochrome P450 to be regenerated after atorvastatin is metabolized first in the liver can be sufficiently ensured.

### Experimental Example 7: Comparative dissolution profiles test

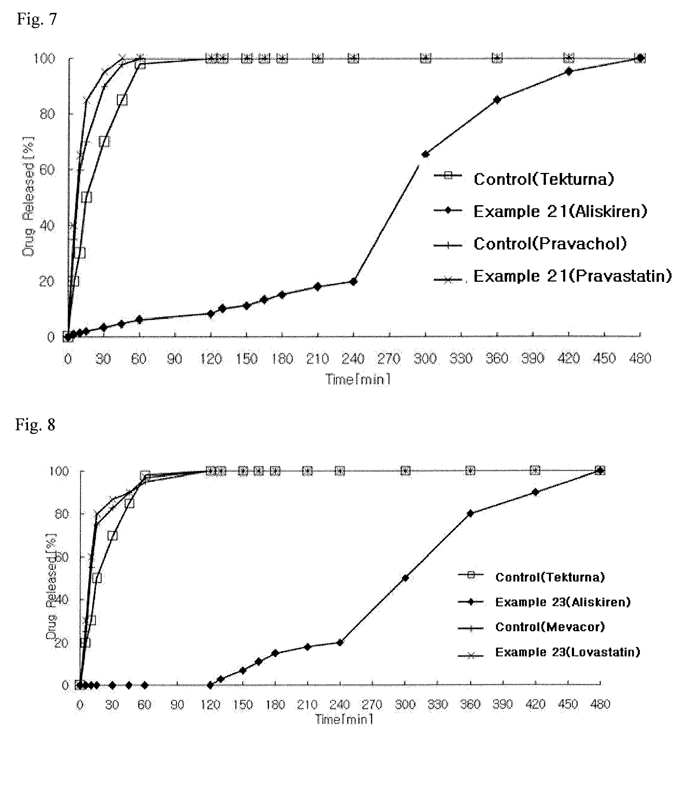

A comparative dissolution profile test was performed using the aliskiren/pravastatin controlled-release capsule preparation (pellet-tablet) prepared in Example 21 and control drugs (Pravachol: pravastatin single drug, Tekturna: aliskiren single drug). The dissolution profile test of the pravastatin ingredient was performed based on the general dissolution test method described in the Korean Pharmacopoeia (8^{th} revision), and the dissolution profile test of the aliskiren ingredient was performed for a total of 480 minutes, in which the dissolution medium was changed from a simulated gastric juice to a dissolution medium of the test method 120 minutes after the start of the test. Details of the dissolution profile test of the pravastatin ingredient are as follows. The results obtained are shown in FIG. 7. Details of the analysis of the aliskiren ingredient are the same as those of Experimental Example 1. FIG. 7 is a graph showing the comparative dissolution profiles of an aliskiren-pravastatin sodium capsule (pellet-tablet) preparation prepared in Example 21, and the aliskiren and pravastatin ingredients of single drugs, Tekturna and Pravachol, as control drugs. In FIG. 7, the x-axis represents the time series, and the y-axis represents the dissolution rate (%).

As can be seen in FIG. 7, when the dissolution profile test was performed under the following conditions, the pravastatin ingredient of the controlled-release capsule preparation (pellet-tablet) of the present invention showed a dissolution profile substantially equal to that of the control drug Pravachol, but the aliskiren ingredient showed a very slow dissolution rate as compared to that of the control drug Tekturna. In the dissolution profile test results for the aliskiren ingredient, the dissolution rate of the aliskiren ingredient up to 120 minutes corresponding to the simulated gastric juice zone was less than 10% in the aliskiren/simvastatin controlled-release capsule preparation (pellet-tablet) of the present invention, but the control drugs showed the completion of release. The dissolution rate of the aliskiren ingredient in the subsequent dissolution medium of the test method zone was 100% in the control formulation, but was about 20% up to a total of 240 minutes in the aliskiren/pravastatin controlled-release capsule preparation (pellet-tablet) of the present invention, which was far lower than that of the control drugs.

As described above, the initial release of aliskiren in the aliskiren/pravastatin controlled-release capsule preparation (pellet-tablet) of the present invention is much slower than pravastatin, unlike dissolution profiles obtained when the aliskiren single drug and the pravastatin single drug, as the control drugs, are administered simultaneously. Thus, in the case of the inventive capsule, the time for metabolism-related enzyme cytochrome P450 to be regenerated after pravastatin is metabolized first in the liver can be sufficiently ensured.

Test Method for pravastatin: Based on the general dissolution test method described in the Korean Pharmacopoeia (8^{th} revision)

Test method: Paddle method, 50 rpm

Dissolution medium: pH 7.0 buffer solution (composition = 0.01 M sodium dihydrogen phosphate solution containing sodium lauryl sulfate 2% wt/wt as surfactant), 900 mL

Analysis method: High performance liquid chromatography

### Experimental Examples 8: Comparative dissolution profile test

A comparative dissolution profile test was performed using the aliskiren/lovastatin capsule preparation (granule-granule) prepared in Example 23 and control drugs (Mevacor: lovastatin single drug, Tekturna: aliskiren single drug). The dissolution profile test of the lovastatin ingredient was performed based on the general dissolution test method described in the Korean Pharmacopoeia (8^{th} revision), and the dissolution profile test of the aliskiren ingredient was performed for a total of 480 minutes, in which the dissolution medium was changed from a simulated gastric juice to a dissolution medium of the test method 120 minutes after the start of the test. Details of the dissolution profile test of the lovastatin ingredient are as follows. The results obtained are shown in FIG. 8. Details of the analysis of the aliskiren ingredient are the same as those of Experimental Example 1. FIG. 8 is a graph showing the comparative dissolution profiles of an aliskiren-lovastatin combination controlled-release capsule (granule-granule) preparation prepared in Example 23, and the aliskiren and lovastatin ingredients of single drugs, Tekturna and Mevacor, as control drugs. In FIG. 8, the x-axis represents the time series, and the y-axis represents the dissolution rate (%).

As can be seen in FIG. 8, when the dissolution profile test was performed under the following conditions, the lovastatin ingredient of the controlled-release capsule preparation (granule-granule) of the present invention showed a dissolution profile substantially equal to that of the control drug Mevacor, but the aliskiren ingredient showed a very slow dissolution rate as compared to that of the control drug Tekturna. In the dissolution profile test results for the aliskiren ingredient, the dissolution rate of the aliskiren ingredient up to 120 minutes corresponding to the simulated gastric juice zone was less than 10% in the aliskiren/lovastatin controlled-release capsule preparation (granule-granule) of the present invention, but the control drugs showed the completion of release. The dissolution rate of the aliskiren ingredient in the subsequent dissolution medium of the test method zone was 100% in the control formulation, but was about 20% up to a total of 240 minutes in the aliskiren/lovastatin controlled-release capsule preparation (granule-granule) of the present invention, which was far lower than that of the control drugs.

As described above, the initial release of aliskiren in the aliskiren/lovastatin controlled-release capsule preparation (granule-granule) of the present invention is much slower than lovastatin, unlike dissolution profiles obtained when the aliskiren single drug and the lovastatin single drug, as the control drugs, are administered simultaneously. Thus, in the case of the inventive capsule, the time for metabolism-related enzyme cytochrome P450 to be regenerated after lovastatin is metabolized first in the liver can be sufficiently ensured.

Test Method for lovastatin: Based on the "Lovastatin tablet" part in United States Pharmacopoeia (USP30)

Test method: Paddle method, 50 rpm

Dissolution medium: pH 7.0 buffer solution (composition = 0.01 M sodium dihydrogen phosphate solution containing sodium lauryl sulfate 2% wt/wt as surfactant), 900 mL

Analysis method: High performance liquid chromatography

**Experimental Example 9: Drug efficacy test (animal test)**

As an experiment to confirm the effects of the invention, an animal test was carried out as follows, in order to confirm the effects of conventional combined administration and inventive chronotherapeutic administration of pharmacologically active ingredients, aliskiren as a renin inhibitor and atorvastatin as an HMG-CoA reductase inhibitor.

### [Animal test]

1) Title: Pharmacokinetic evaluation of aliskiren and atorvastatin in a hypertensive animal model.
2) Object: It is intended to verify through the chronotherapeutic administration-based pharmacokinetic changes the fact that the problem of pharmacokinetic interaction between different drugs upon simultaneous administration of drug groups metabolized by the same hepatic enzyme can be solved by controlled release of the drugs.
3) Test animal: SD rats
4) Test method:
   1. Test group: Animals were grouped into two groups (n=6), a group receiving simultaneous administration of aliskiren/atorvastatin, and a group receiving chronotherapeutic administration of aliskiren/atorvastatin.
   2. Administration route: Forced intragastric administration via an oral sonde.
   3. Administration method:
      - Simultaneous administration group: aliskiren and atorvastatin were simultaneously administered.
      - Chronotherapeutic administration group: aliskiren was administered at a time-lag interval of 4 hours after administration of atorvastatin.
   4. Administration dose:
      - Aliskiren: 300 mg/kg
      - Atorvastatin: 80 mg/kg
   5. Administration volume: 5mL/kg based on the body weight of animals measured on the day of administration.
   6. Administration frequency: Once a day for 8 days at the same time point of administration, for a total of 8 times.
   7. Blood collection:
      - Simultaneous administration group: Blood was collected on Day 8 of administration, and post-administration 0.33, 0.67, 1, 2, 4, 4.33, 4.67, 5, 6, 8, 10, 12, 16, 24, 28, 48, 52, 72 and 76 hours (total of 19 times) (200 mL in terms of blood plasma)
      - Chronotherapeutic administration group: Blood was collected on Day 8 of administration, and post-administration 0.33, 0.67, 1, 2, 4, 8, 12, 24, 48 and 72 hours (total of 10 times) (200 mL in terms of blood plasma)
   8. Analysis
      - Measurement items: Cₘₐₓ, AUC
      - Analysis method: Analysis was carried out using LC/MS/MS, with reference to an analysis method of single agents.

From the results of an animal test, the simultaneous administration group exhibited a significant increase in blood level of aliskiren, as compared to the single administration group. On the other hand, the chronotherapeutic administration group exhibited the blood aliskiren level equivalent to that of the single administration group, even with a combination of different drugs which are metabolized by the same enzyme system.

Accordingly, it can be seen that through the delayed-release preparation of the present invention designed based on xenobiotics and chronotherapy, it is possible to solve the problems associated with the risk that intrinsic therapeutic effects of drugs cannot be obtained due to pharmacokinetic changes of specific ingredients resulting from the interaction between different drugs upon simultaneous administration thereof, and further side effects may be expressed.

### INDUSTRIAL APPLICABILITY

The present invention provides a pharmaceutical preparation including a compartment containing a renin inhibitor as a pharmacologically active ingredient, and a compartment containing an HMG-CoA reductase inhibitor as a pharmacologically active ingredient, wherein one compartment is a prior-release compartment and the other compartment is a delayed-release compartment. The combination preparation of the present invention can deliver a renin inhibitor and an HMG-CoA reductase inhibitor with a time interval at a specific speed, thus reducing undesirable side-effects, improving the drug efficacy and promoting the patient compliance. Further, the pharmaceutical preparation of the present invention has pharmacological, clinical, scientific and economical advantages in the prevention or treatment of metabolic syndromes, cardiovascular diseases, renal diseases and the like, as compared with the complex drug regimens in which medicament ingredients are taken individually or simultaneously.

## Claims

1. A pharmaceutical preparation comprising a compartment containing a renin inhibitor as a pharmacologically active ingredient, and a compartment containing a 3-hydroxy-3-methylglutaryl coenzyme A (HMG-CoA) reductase inhibitor as a pharmacologically active ingredient, wherein one compartment is a prior-release compartment and the other compartment is a delayed-release compartment.

2. The pharmaceutical preparation according to claim 1, wherein the renin inhibitor is selected from aliskiren, remikiren, enalkiren, zankiren, detikiren, terlakiren, isomers thereof or pharmaceutically acceptable salts thereof.

3. The pharmaceutical preparation according to claim 1, wherein the HMG-CoA reductase inhibitor is selected from atorvastatin, simvastatin, pitavastatin, rosuvastatin, fluvastatin, pravastatin, lovastatin, isomers thereof or pharmaceutically acceptable salts thereof.

4. The pharmaceutical preparation according to claim 1, wherein the renin inhibitor is aliskiren, an isomer thereof or a pharmaceutically acceptable salt thereof and the HMG-CoA reductase inhibitor is atorvastatin, an isomer thereof or a pharmaceutically acceptable salt thereof.

5. The pharmaceutical preparation according to claim 1, wherein the renin inhibitor is aliskiren, an isomer thereof or a pharmaceutically acceptable salt thereof and the HMG-CoA reductase inhibitor is simvastatin, an isomer thereof or a pharmaceutically acceptable salt thereof.

6. The pharmaceutical preparation according to claim 1, wherein the renin inhibitor is aliskiren, an isomer thereof or a pharmaceutically acceptable salt thereof and the HMG-CoA reductase inhibitor is fluvastatin, an isomer thereof or a pharmaceutically acceptable salt thereof.

7. The pharmaceutical preparation according to claim 1, wherein the renin inhibitor is aliskiren, an isomer thereof or a pharmaceutically acceptable salt thereof and the HMG-CoA reductase inhibitor is rosuvastatin, an isomer thereof or a pharmaceutically acceptable salt thereof.

8. The pharmaceutical preparation according to claim 1, wherein the renin inhibitor is aliskiren, an isomer thereof or a pharmaceutically acceptable salt thereof and the HMG-CoA reductase inhibitor is pitavastatin, an isomer thereof or a pharmaceutically acceptable salt thereof.

9. The pharmaceutical preparation according to claim 1, wherein the renin inhibitor is aliskiren, an isomer thereof or a pharmaceutically acceptable salt thereof and the HMG-CoA reductase inhibitor is pravastatin, an isomer thereof or a pharmaceutically acceptable salt thereof.

10. The pharmaceutical preparation according to claim 1, wherein the renin inhibitor is aliskiren, an isomer thereof or a pharmaceutically acceptable salt thereof and the HMG-CoA reductase inhibitor is lovastatin, an isomer thereof or a pharmaceutically acceptable salt thereof.

11. The pharmaceutical preparation according to any one of claims 1 to 10, wherein a pharmacologically active ingredient of the prior-release compartment is released at a level of more than 80% by weight of a total amount of the pharmacologically active ingredient in the preparation within one hour after the release of the pharmacologically active ingredient is initiated.

12. The pharmaceutical preparation according to any one of claims 1 to 10, wherein a pharmacologically active ingredient of the delayed-release compartment is released at a level of less than 20% by weight of a total amount of the pharmacologically active ingredient of the delayed-release compartment by 2 hours after the release of the pharmacologically active ingredient of the prior-release compartment is initiated.

13. The pharmaceutical preparation according to any one of claims 1 to 10, wherein a content of the HMG-CoA reductase inhibitor is 1 to 3000 parts by weight, relative to 100 parts by weight of the renin inhibitor.

14. The pharmaceutical preparation according to any one of claims 1 to 10, wherein a content of the renin inhibitor in the pharmaceutical preparation is in the range of 10 to 1000 mg.

15. The pharmaceutical preparation according to any one of claims 1 to 10, wherein a content of the HMG-CoA reductase inhibitor in the pharmaceutical preparation is in the range of 0.5 to 160 mg.

16. The pharmaceutical preparation according to any one of claims 1 to 10, wherein the delayed-release compartment further includes at least one release-controlling material selected from an enteric polymer, a water-insoluble polymer, a hydrophobic compound, a hydrophilic polymer and a mixture thereof, in addition to pharmacologically active ingredients.

17. The pharmaceutical preparation according to claim 16, wherein a content of the release-controlling material is in the range of 0.01 to 100 parts by weight, relative to 1 part by weight of the pharmacologically active ingredient.

18. The pharmaceutical preparation according to claim 16, wherein the enteric polymer is at least one selected from the group consisting of an enteric cellulose derivative, an enteric acrylic acid copolymer, an enteric maleic acid copolymer, an enteric polyvinyl derivative, and a mixture thereof.

19. The pharmaceutical preparation according to claim 18, wherein the enteric cellulose derivative is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid copolymer, a methacrylic acid/methyl methacrylate copolymer, a methacrylic acid/ethyl acrylate copolymer, a methyl acrylate/methacrylic acid/octyl acrylate copolymer and a mixture thereof; the enteric maleic acid copolymer is at least one selected from a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetal phthalate, polyvinylbutyrate phthalate, polyvinylacetacetal phthalate and a mixture thereof.

20. The pharmaceutical preparation according to claim 19, wherein a content of the enteric polymer is in the range of 0.1 parts by weight to 20 parts by weight, relative to 1 part by weight of the pharmacologically active ingredient.

21. The pharmaceutical preparation according to claim 16, wherein the water-insoluble polymer is at least one selected from the group consisting of polyvinyl acetate, a polymethacrylate copolymer, a poly(ethyl acrylate, methyl methacrylate) copolymer, a poly(ethyl acrylate, methyl methacrylate, trimethylaminoethyl methacrylate) copolymer, ethylcellulose, cellulose ester, cellulose ether, cellulose acylate, cellulose diacylate, cellulose triacylate, cellulose acetate, cellulose diacetate, cellulose triacetate and a mixture thereof.

22. The pharmaceutical preparation according to claim 21, wherein a content of the water-insoluble polymer is in the range of 0.1 parts by weight to 30 parts by weight, relative to 1 part by weight of the pharmacologically active ingredient.

23. The pharmaceutical preparation according to claim 21, wherein the water-insoluble polymer is at least one selected from the group consisting of polyvinylacetate, a poly(ethyl acrylate, methyl methacrylate, trimethylaminoethyl methacrylate) copolymer, ethyl cellulose, cellulose acetate and a mixture thereof.

24. The pharmaceutical preparation according to claim 16, wherein the release-controlling material is at least one selected from an enteric polymer, a hydrophilic polymer and a water-insoluble polymer.

25. The pharmaceutical preparation according to claim 24, wherein the release-controlling material is at least one selected from a water-insoluble polymer, hydroxypropylcellulose, hydroxypropylmethylcellulose, hydroxypropylmethylcellulose phthalate, a methacrylic acid/ethyl acrylate copolymer, and a carboxyvinyl polymer.

26. The pharmaceutical preparation according to claim 16, wherein the hydrophobic compound is at least one selected from a fatty acid or fatty acid ester, a fatty acid alcohol, a wax, an inorganic material, and a mixture thereof.

27. The pharmaceutical preparation according to claim 26, wherein the fatty acid or fatty acid ester is at least one selected from glyceryl palmitostearate, glyceryl stearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof.

28. The pharmaceutical preparation according to claim 26, wherein a content of the hydrophobic compound is in the range of 0.1 parts by weight to 20 parts by weight, relative to 1 part by weight of the pharmacologically active ingredient.

29. The pharmaceutical preparation according to claim 26, wherein the hydrophilic polymer is at least one selected from saccharide, a cellulose derivative, gum, a protein, a polyvinyl derivative, a polymethacrylate copolymer, a polyethylene derivative, a carboxyvinyl copolymer and a mixture thereof.

30. The pharmaceutical preparation according to claim 29, wherein the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose, hydroxypropylcellulose, hydroxymethylcellulose, hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the polymethacrylate copolymer is at least one selected from a poly(butyl methacrylate, (2-dimethylaminoethyl) methacrylate, methyl methacrylate) copolymer, a poly(methacrylic acid, methyl methacrylate) copolymer, a poly(methacrylic acid, ethyl acrylate) copolymer and a mixture thereof; the polyethylene derivative is at least one selected from polyethylene glycol, polyethylene oxide and a mixture thereof; and the carboxyvinyl polymer is carbomer.

31. The pharmaceutical preparation according to claim 29, wherein a content of the hydrophilic polymer is in the range of 0.05 parts by weight to 30 parts by weight, relative to 1 part by weight of the pharmacologically active ingredient.

32. The pharmaceutical preparation according to any one of claims 1 to 10, wherein the pharmaceutical preparation is in the form of a two-phase matrix tablet which is obtained by uniformly mixing a delayed-release compartment and a prior-release compartment, followed by compression.

33. The pharmaceutical preparation according to any one of claims 1 to 10, wherein the pharmaceutical preparation is in the form of a film-coated tablet including a tablet consisting of a delayed-release compartment and a film-coated layer consisting of a prior-release compartment enclosing the exterior of the tablet.

34. The pharmaceutical preparation according to any one of claims 1 to 10, wherein the pharmaceutical preparation is in the form of a multi-layered tablet having a multi-layered structure of a delayed-release compartment and a prior-release compartment.

35. The pharmaceutical preparation according to any one of claims 1 to 10, wherein the pharmaceutical preparation is in the form of a press coated tablet including an inner core tablet consisting of a delayed-release compartment and an outer layer consisting of a prior-release compartment enclosing the outer surface of the inner core tablet.

36. The pharmaceutical preparation according to claim 35, wherein the press coated tablet is an osmotic press coated tablet.

37. The pharmaceutical preparation according to any one of claims 1 to 10, wherein the pharmaceutical preparation is in the form of a capsule including a particle, granule, pellet, or tablet consisting of a delayed-release compartment and a particle, granule, pellet, or tablet consisting of a prior-release compartment.

38. The pharmaceutical preparation according to any one of claims 1 to 10, further comprising a coating layer on the outside of the delayed-release compartment and/or the prior-release compartment.

39. The pharmaceutical preparation according to any one of claims 1 to 10, wherein the delayed-release compartment is a compartment which contains an osmo-regulator and is coated by a semi-permeable membrane coating base.

40. The pharmaceutical preparation according to claim 39, wherein the osmo-regulator is at least one selected from the group consisting of magnesium sulfate, magnesium chloride, sodium chloride, lithium chloride, potassium sulfate, sodium sulfate, lithium sulfate and a mixture thereof.

41. The pharmaceutical preparation according to claim 39, wherein the semi-permeable membrane coating base is at least one selected from the group consisting of polyvinyl acetate, a polymethacrylate copolymer, a poly(ethyl acrylate, methyl methacrylate) copolymer, a poly(ethyl acrylate, methyl methacrylate, trimethylaminoethyl methacrylate) copolymer, ethylcellulose, cellulose ester, cellulose ether, cellulose acylate, cellulose diacylate, cellulose triacylate, cellulose acetate, cellulose diacetate, cellulose triacetate and a mixture thereof.

42. The pharmaceutical preparation according to any one of claims 1 to 10, wherein the pharmaceutical preparation is in the form of a coated tablet further including a coating layer on the outside thereof.

43. The pharmaceutical preparation according to any one of claims 1 to 10, wherein the pharmaceutical preparation is in the form of a kit including a delayed-release compartment and a prior-release compartment.

44. The pharmaceutical preparation according to any one of claims 1 to 10, wherein the pharmaceutical preparation is for evening administration.

45. A method for preventing and treating at least one disease selected from a metabolic syndrome, a cardiovascular diseases and a kidney disease, comprising administering a pharmaceutical preparation of any one of claims 1 to 10 to a mammal including a human.
